# EUROPEAN PATENT SPECIFICATION

(11) **EP 4 302 753 B1**
(45) Date of publication and mention of the grant of the patent: **02.09.2026**
(21) Application number: 22763278.3
(22) Date of filing: 01.03.2022
(51) Int. Cl.: A61K 9/10, A61K 47/32, A61K 47/34, B01J 13/12, A61K 35/14

(54) **MICROPARTICLES AND MICROPARTICLE DISPERSION**
MIKROPARTIKEL UND MIKROPARTIKELDISPERSION
MICROPARTICULES ET DISPERSION DE MICROPARTICULES

(30) Priority: 01.03.2021 JP 2021032109
(43) Date of publication of application: 10.01.2024
(73) Proprietor: The University of Tokyo, Bunkyo-ku, Tokyo 113-8654 (JP); DAIKIN INDUSTRIES, LTD., Osaka-shi, Osaka 530-0001 (JP)
(72) Inventor: ITO, Taichi, Tokyo 113-8654 (JP); CHANDEL, Arvind Kumar Singh, Tokyo 113-8654 (JP); HAMADA, Tomohito, Osaka-Shi, Osaka 530-0001 (JP); SAGISAKA, Shigehito, Osaka-Shi, Osaka 530-0001 (JP); KISHIKAWA, Yosuke, Osaka-Shi, Osaka 530-0001 (JP)
(74) Representative: Hoffmann Eitle
(86) International application number: PCT/JP2022/008668
(87) International publication number: WO 2022/186224

(56) References cited:
- JP-A- 2016 503 100
- JP-A- H06 507 830
- JP-A- H07 216 127
- US-A- 5 374 243
- US-A1- 2008 008 657
- US-A1- 2010 221 190
- US-A1- 2017 348 401
- DATABASE WPI Week 2021032, Derwent World Patents Index; AN 2021-33039V, XP002812213

## Description

### Technical Field

The present disclosure relates to a microparticle and a microparticle dispersion.

### Background Art

Micro-sized particles that can be used as oxygen carriers have been developed (Non-Patent Literature 1 and 2). Such particles can be used as an alternative to red blood cells, and the dispersion of the particles can be used as an alternative to blood.

As such microparticles, for example, hydrogel-immobilized microparticles having a diameter of 6 µm, which mimic the size and disc shape of red blood cells and have deformability, have been reported (Non-patent Literature 3).

US 2010/0221190 A1 describes a method for the production of particles comprising a gas core and a shell which method comprises the steps of: a) providing a mixture comprising a shell composition, a first solvent (1) and a second non-solvent (2); b) combining the mixture of step (a) with an aqueous composition thereby forming an emulsion of the mixture of step (a) in an aqueous phase; c) applying conditions for volatizing solvent (1); d) applying conditions for removal of water; e) applying conditions for removing of non-solvent (2), wherein the non-solvent (2) is selected from the group comprising organic compositions that have a vapor pressure significantly lower than water under the conditions of step (d).

US 2017/0348401 A1 describes a pharmaceutical formulation comprising an aqueous suspension of gas-filled microvesicles with a stabilizing envelope, said stabilizing envelope comprising (a) an antigen covalently bound to an amphiphilic component forming said stabilizing envelope, (b) a phospholipid, and (c) a fatty acid, with a molar ratio between the phospholipid and the fatty acid lower than 30/70.

US 2008/0008657 A1 describes a composition for diagnostic and/or therapeutic imaging which comprises a mixture of at least two different preparations of gas-filled microvesicles, wherein said at least two different preparations have respective peaks of non-linear echographic response differing by at least 2 MHz to each other.

WO 2021/060303 A1 describes a core-shell type microparticles containing a shell containing a polymer having a Young's modulus of 100 MPa or less and a core containing fluorocarbon.

### Citation List

### Non-patent Literature

NPL 1: Yu Xiong, et al., Biomacromolecules (13) 3292-3300, 2012
NPL 2: Yu Xiong, et al., ACSNANO (7) 7454-7461, 2013
NPL 3: Chen K, et al., Biomacromolecules (13) 2748-2759, 2012

### Summary of Invention

### Technical Problem

An object of the present disclosure is to provide a novel microparticle and a novel microparticle dispersion.

### Solution to Problem

The present invention is defined in the appended claims.

The present invention provides a core-shell microparticle comprising
a shell comprising at least one polymer selected from the group consisting of fluorine-containing polymers, fluorine-free vinyl polymers, and silicone polymers, and
a core comprising a fluorocarbon, wherein the fluorocarbon is in liquid or gel form.

Preferably, the structural unit constituting the main chain in the repeating unit in the main chain of the polymer substantially comprises at least one structural unit selected from the group consisting of -CX¹X²-CX³CX⁴-, -CX¹=CX²-, -CX¹-O-CX²-, -CX¹X²-C(=O)-CX³X⁴-, -C(=O)-N(X,Y)-, -CX¹X²-C(=S)-CX¹X²-, -Si(X,Y)-O-Si(X,Y)-, and -Si(X,Y)-Si(X,Y)-, wherein X¹, X², X³, and X⁴ are the same or different and each represents a hydrogen atom, a fluorine atom, a chlorine atom, an optionally substituted oxygen atom, an optionally substituted chain or cyclic C₁₋₂₀ alkyl group, an optionally substituted C₁₋₂₀ alkylene group, a nitrile group, an ester group, an optionally substituted C₁₋₂₀ fluorine-containing alkyl group, or an optionally substituted C₁₋₂₀ fluorine-containing alkylene group, and X and Y are the same or different and each represents a hydrogen atom, an optionally substituted chain or cyclic C₁₋₂₀ alkyl group, an optionally substituted chain or cyclic C₁₋₂₀ alkylene group, an optionally substituted chain or cyclic C₁₋₂₀ aryl group, an optionally substituted chain or cyclic C₁₋₂₀ (per)fluoroalkyl group, or an optionally substituted chain or cyclic C₁₋₂₀ (per) fluoroalkylene group.

Preferably, the polymer has a change in tensile strength after application of an acidic condition of 50% or less.

Preferably, the polymer has a change in tensile elongation after application of an acidic condition of 50% or less.

Preferably, the polymer has a change in tensile strength after application of an alkaline condition of 50% or less.

Preferably, the polymer has a change in tensile elongation after application of an alkaline condition of 50% or less.

Preferably, the microparticle has a shape-change ratio after application of an acidic condition of 50% or less.

Preferably, the microparticle has a shape-change ratio after application of an alkaline condition of 50% or less.

Preferably, the shell has an oxygen permeability coefficient of 10⁻¹⁴ cm³ (STP) cm/ (cm²·s·Pa) or more.

Preferably, the polymer is a silicone polymer or a fluorine-containing polymer, and the fluorine content in the polymer is 20 wt% or more.

Preferably, the fluorocarbon in the core has a fluorine content of 10 mass% or more.

Preferably, the fluorocarbon in the core has a boiling point of 90°C or more.

Preferably, the fluorocarbon in the core has an oxygen solubility of 30 vol/vol% or more.

Preferably, the fluorocarbon is a fluorocarbon containing a perfluoroalkyl group or a fluorocarbon containing a fluoropolyether group and a perfluoroalkyl group, and the fluorocarbon has a weight average molecular weight of 50,000 or less.

Preferably, the fluorocarbon containing a fluoropolyether group and a perfluoroalkyl group has a boiling point of 150°C or more.

Preferably, the microparticle has a volume average particle diameter of 500 µm or less.

Preferably, the shell has a thickness of 50 nm or more. Preferably, the core contains oxygen.

Preferably, the shell has an elastic modulus of 500 MPa or less. The present invention also provides a composition comprising the microparticle according to the present invention and an organic solvent.

The present invention also provides a microparticle dispersion comprising the microparticle according to the present invention and an aqueous medium, wherein the microparticle is dispersed in the aqueous medium.

Preferably, the microparticle dispersion is an oxygen-carrying solution, a transplanted tissue preservation solution, a transplanted organ protection solution, or a culture solution.

The present invention also provides a method for producing the microparticle according to the present invention, comprising the following steps (1) and (2):
(1) the step of subjecting a dispersed phase in which the polymer and the fluorocarbon are dispersed in an organic solvent to membrane emulsification in a continuous phase containing a surfactant via a porous membrane having a uniform pore diameter, thus obtaining an emulsion containing the microparticle as a dispersoid, and
(2) the step of removing an organic solvent phase contained in the emulsion.

The present invention also provides a method for producing the microparticle according to the present invention having a dent, comprising the following steps (1) and (2):
(1) the step of adding an alcohol to a dispersion obtained by dispersing the microparticle according to the invention in an aqueous medium, and
(2) the step of resuspending the particle obtained in step (1) in a solvent.

### Advantageous Effects of Invention

According to the present disclosure, it is possible to provide a novel microparticle and a novel microparticle dispersion.

### Brief Description of Drawings

In Fig. 1, Fig. 1A is a schematic cross-sectional view showing a core-shell type spherical microparticle according to one embodiment of the present disclosure; and Fig. 1B is a schematic cross-sectional view showing a microparticle according to a modified embodiment.
In Fig. 2, Fig. 2A is a schematic cross-sectional view showing a core-shell microparticle having a dent according to one embodiment of the present disclosure; and Fig. 2B is a schematic upper cross-sectional view showing a core-shell microparticle having a dent according to one embodiment of the present disclosure.
Fig. 3 is a schematic diagram showing a manufacturing apparatus used in a method for producing a microparticle according to one embodiment of the present disclosure.
Fig. 4 is a drawing illustrating the principle of the membrane emulsification method via a porous membrane. Description of Embodiments

### 1. Microparticle

The microparticle of the present invention is a core-shell microparticle comprising a shell containing a polymer selected from fluorine-containing polymers, fluorine-free vinyl polymers, and silicone polymers, and a core containing a fluorocarbon, which is in liquid or gel form.

In the present disclosure, the "microparticle" generally refers to a particle having a diameter of less than 1000 µm, and also includes a particle having a diameter of less than 1 µm.

The microparticle of the present disclosure can be used in various applications including an oxygen carrier. The size of the microparticle of the present disclosure can be suitably determined according to applications, and an optimum size for each application is well-known to those skilled in the art.

When the microparticle of the present disclosure is particularly used as an oxygen carrier, the volume average particle diameter is preferably 500 µm or less, more preferably 100 µm or less, still more preferably 50 µm or less, and even more preferably 20 µm or less. The microparticle of the present disclosure preferably has a volume average particle diameter of 100 nm or more, more preferably 500 nm or more, still more preferably 1 µm or more, and even more preferably 2 µm or more, in terms of being less susceptible to endocytosis of cells. In the present disclosure, the volume average particle diameter is measured by a scanning electron microscope image.

The microparticles of the present disclosure preferably have a controlled size and/or uniform particle size distribution. The microparticles of the present disclosure preferably have a monodisperse size distribution. The CV value of the microparticles of the present disclosure is preferably 70% or less, more preferably 40% or less, still more preferably 35% or less, and even more preferably 30% or less. In the present disclosure, the CV value of microparticles is a coefficient of variation of particle size distribution determined by a standard deviation of a particle size and a volume average particle diameter. The lower the CV value, the more excellent the uniformity of the particle size. Specifically, the coefficient of variation (CV value) of particle size distribution can be calculated by the following equation. 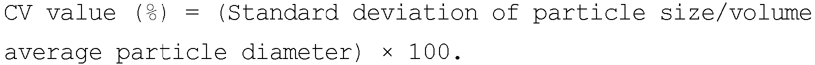 CV value (%) = (Standard deviation of particle size/volume average particle diameter) × 100.

The shape of the microparticle of the present disclosure is not limited, and may be a spherical shape, a non-spherical shape, an elliptical shape (oblong spherical shape), a rod shape, a pyramidal shape, a cubic shape, a disk shape, or a shape having a dent (concave shape). When the surface shape displacement of a disc-shaped particle from the center of the circle toward the stereocenter is observed, it can be determined that the disc-shaped particle has a concave shape, i.e., a dent. The microparticle of the present disclosure may be spherical or have a dent. The microparticle of the present disclosure preferably have a dent particularly from the viewpoint of easily passing through a narrow tube having a size of about 1 to 50 µm, such as a capillary or a microchannel. When the microparticle has a dent, it is easily deformed into a slipper shape and a parachute shape, as in blood cells, and easily passes through a narrow tube. In this case, the shape with a dent is preferably a shape close to a red blood cell, and specifically is a concaved shape. The microparticle having a dent can further increase the specific surface area, which can increase the gas permeability of a gas such as oxygen.

The thickness of the shell of the microparticle of the present disclosure is not limited, and it is, for example, 0.05 µm or more, 0.1 µm or more, or 0.5 µm or more from the viewpoint of stability of particles. The thickness of the shell is preferably small as long as stability is ensured, and is, for example, 4 µm or less. The thickness of the shell of the microparticle is preferably, for example, 0.1 to 4 µm. The thickness of the shell of the microparticle may be, for example, 0.2 to 4 µm or 0.5 to 4 µm. In the present disclosure, the thickness of the shell is measured from a photograph of cross section using a confocal microscope or an electron microscope.

The microparticle of the present disclosure has a core-shell structure. The core-shell microparticle is different from a conventional emulsion-type artificial oxygen carrier and a non-encapsulated type artificial oxygen carrier in which a component that occludes oxygen, such as a fluorocarbon, is present on the particle surface. The core-shell microparticle stably holds the fluorocarbon inside, and is excellent in stability as compared with emulsion type or non-encapsulated type oxygen carriers.

The microparticle of the present disclosure preferably has an elastic modulus of 1 to 5000 MPa, more preferably 5 to 3000 MPa, and even more preferably 10 to 2000 MPa.

The microparticle of the present disclosure may be sterile. Examples of the sterilization method include ethanol sterilization, ethylene oxide sterilization, UV sterilization, electron beam (y) sterilization, ozone sterilization, autoclave, dry heat sterilization, glutaraldehyde sterilization, and orthophthalaldehyde sterilization. In the case of sterilization, it is preferable that the shape of the core-shell type particle does not change even after exposure to the conditions; and when the shape of the core-shell type particle changes, the change rate is preferably 50% or less, more preferably 30% or less, and even more preferably 10% or less.

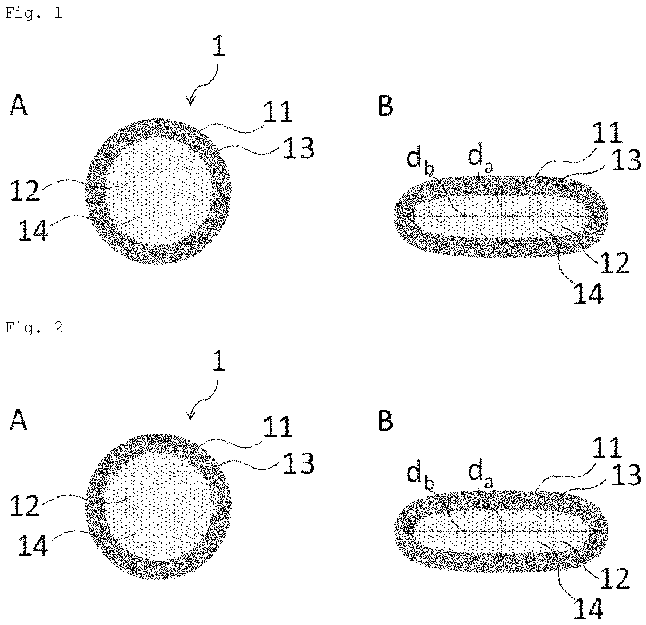

Fig. 1A is a schematic cross-sectional view showing a core-shell type spherical microparticle according to one embodiment of the present disclosure. Fig. 1B is a schematic cross-sectional view showing a microparticle of a modified embodiment. Figs. 2A and 2B are schematic cross-sectional views each showing a core-shell microparticle having a dent according to one embodiment of the present disclosure. Fig. 2A is a cross-sectional view of a microparticle, and Fig. 2B is an upper cross-sectional view of a microparticle. As shown in Figs. 1 and 2, the microparticle 1 is a microcapsule having a shell 11 and a core 12. The core 12 forms the inside of the microparticle 1 and is enclosed or surrounded by the shell 11 forming the outside of the microparticle 1.

The microparticle shown in Fig. 1A has a spherical shape. The microparticle 1 shown in Fig. 2 has a substantially disc shape and has a dent 15 in the central portion.

The core 12 includes a fluorocarbon 14. The fluorocarbon 14 has excellent gas (e.g., oxygen) solubility. Accordingly, the microparticle 1 can function as a carrier capable of holding a gas in the core and transporting the gas to the target site/tissue. The microparticle according to one embodiment can function as a gas carrier having deformation permeability of a red blood cell or the like.

The shell 11 comprises a polymer 13. The shell 11 containing the polymer 13 can provide a deformable microparticle 1, which has excellent elasticity, deformability, and flexibility. In the present specification, "deformable" means that the core-shell structure can be maintained even when the particle is deformed. In the microparticle according to one embodiment, the shell does not collapse even when the particle is deformed, and the core component is encapsulated inside the shell or surrounded by the shell. For example, the microparticle can be deformed by compression with a displacement of 25% or more of the particle size (for example, 30% or more or 35% or more). For example, as shown in Fig. 1B, the spherical microparticle of Fig. 1A may be deformed into an elongated spherical microparticle having a short diameter dₐ and a long diameter d_{b}.

### 1.1 Shell

The microparticle of the present invention comprises a shell, and the shell comprises a specific polymer. The polymer of the present invention comprises at least one polymer selected from the group consisting of fluorine-containing polymers, fluorine-free vinyl polymers, and silicone polymers. The polymers may be used alone or in a combination of two or more. The fluorine-containing polymer refers to a polymer containing a fluorine atom in the structural formula. The fluorine-free vinyl polymer refers to a polymer that does not contain a fluorine atom in the structural formula that is derived from a monomer containing a vinyl group. The silicone polymer refers to a polymer that does not contain a fluorine atom in the structural formula and contains a silicon-silicon bond or a silicon-oxygen bond.

In an aspect, the polymer of the present disclosure is a polymer in which the structural unit constituting the main chain in the repeating unit in the main chain of the polymer substantially comprises at least one structural unit selected from the group consisting of -CX¹X²-CX³CX⁴-, -CX¹=CX²-, -C≡C-, -CX¹-O-CX²-, -CX¹-S-CX²-, -CX¹-S-S-CX²-, -CX¹X²-C(=O)-CX³X⁴--, -C(=O)-N(X,Y)-, -CX¹X²-C(=S)-CX¹X²-, -C(=S)-N(X,Y)-, -Si(X,Y)-O-Si(X,Y)-, and -Si(X,Y)-Si(X,Y)-, wherein X¹, X², X³, and X⁴ are the same or different and each represents a hydrogen atom, a fluorine atom, a chlorine atom, an optionally substituted oxygen atom, an optionally substituted chain or cyclic C₁₋₂₀ alkyl group, an optionally substituted C₁₋₂₀ alkylene group, a nitrile group, an ester group, an optionally substituted C₁₋₂₀ fluorine-containing alkyl group, or an optionally substituted C₁₋₂₀ fluorine-containing alkylene group, and X and Y are the same or different and each represents a hydrogen atom, an optionally substituted chain or cyclic C₁₋₂₀ alkyl group, an optionally substituted chain or cyclic C₁₋₂₀ alkylene group, an optionally substituted chain or cyclic C₁₋₂₀ aryl group, an optionally substituted chain or cyclic C₁₋₂₀ (per)fluoroalkyl group, or an optionally substituted chain or cyclic C₁₋₂₀ (per) fluoroalkylene group). In this case, the polymer of the present disclosure does not contain a hydrolyzable functional group in the main chain, and thus has excellent stability in water. Since the shell is formed of such a polymer, the microparticle of the present disclosure has excellent stability in water. Due to having the stability in water, the shell allows the core material to be stably encapsulated in the formation of the core-shell particle, the polymer of the present disclosure provides stability when dispersed in water as a core-shell particle because. In the present disclosure, "a polymer in which the structural unit constituting the main chain in the repeating unit in the main chain substantially comprises a specific structural unit" includes a case where the structural unit constituting the main chain substantially consists of the specific structural unit. In this case, "substantially" means that the structural unit constituting the main chain may include other structural units as long as an effect of making the microparticle of the present disclosure excellent in stability in water can be obtained. In the present disclosure, the term "a polymer in which the structural unit constituting the main chain in the repeating unit in the main chain substantially comprises a specific structural unit" specifically means that "the structural unit constituting the main chain in the repeating unit in the main chain contains 90 mass% or more, preferably 95 mass% or more, more preferably 97 mass% or more, still more preferably 98 mass% or more, and even more preferably 99 mass% or more of the specific structural unit in total with respect to the structural unit constituting the main chain." In the present disclosure, the description "a polymer in which the structural unit constituting the main chain in the repeating unit in the main chain substantially comprises a specific structural unit" includes a case where the structural unit constituting the main chain includes only the specific structural unit.

In the polymer of the present disclosure, it is further preferable that the structural unit constituting the main chain in the repeating unit in the main chain of the polymer is substantially at least one structural unit selected from the group consisting of -CX¹X²CX³CX⁴-, -CX¹=CX²-, -CX¹-O-CX²-, -CX¹X²-C(=O)-CX³X⁴--, -C(=O)-N(X,Y)-, -CX¹X²-C(=S)-CX¹X²-, -Si(X,Y)-O-Si(X,Y)-, and -Si(X,Y)-Si(X,Y)-, wherein X¹, X², X³, and X⁴ are the same or different and each represents a hydrogen atom, a fluorine atom, a chlorine atom, an optionally substituted oxygen atom, an optionally substituted chain or cyclic C₁₋₂₀ alkyl group, an optionally substituted C₁₋₂₀ alkylene group, a nitrile group, an ester group, an optionally substituted C₁₋₂₀ fluorine-containing alkyl group, or an optionally substituted C₁₋₂₀ fluorine-containing alkylene group, and X and Y are the same or different and each represents a hydrogen atom, an optionally substituted chain or cyclic C₁₋₂₀ alkyl group, an optionally substituted chain or cyclic C₁₋₂₀ alkylene group, an optionally substituted chain or cyclic C₁₋₂₀ aryl group, an optionally substituted chain or cyclic C₁₋₂₀ (per)fluoroalkyl group, or an optionally substituted chain or cyclic C₁₋₂₀ (per) fluoroalkylene group).

X¹, X², X³, and X⁴ are preferably the same or different and each represents a hydrogen atom, a fluorine atom, a chlorine atom, an optionally substituted oxygen atom, an optionally substituted chain or cyclic C₁₋₁₆ alkyl group, an optionally substituted C₁₋₁₆ alkylene group, a nitrile group, an ester group, an optionally substituted C₁₋₁₆ fluorine-containing alkyl group, an optionally substituted C₁₋₁₆ fluorine-containing alkylene group, and X and Y are the same or different and each represents a hydrogen atom, an optionally substituted chain or cyclic C₁₋₁₆ alkyl group, an optionally substituted chain or cyclic C₁₋₁₆ alkylene group, an optionally substituted chain or cyclic C₁₋₁₆ aryl group, an optionally substituted chain or cyclic C₁₋₁₆ (per)fluoroalkyl group, an optionally substituted chain or cyclic C₁₋₁₆ (per) fluoroalkylene group. More preferably, X¹, X², X³, and X⁴ are the same or different and each represents a hydrogen atom, a fluorine atom, a chlorine atom, an optionally substituted oxygen atom, an optionally substituted chain or cyclic C₁₋₈ alkyl group, an optionally substituted C₁₋₈ alkylene group, a nitrile group, an optionally substituted C₁₋₈ fluorine-containing alkyl group, or an optionally substituted C₁₋₈ fluorine-containing alkylene group, and X and Y are the same or different and each represents a hydrogen atom, an optionally substituted chain or cyclic C₁₋₈ alkyl group, an optionally substituted chain or cyclic C₁₋₈ alkylene group, an optionally substituted chain or cyclic C₁₋₈ aryl group, an optionally substituted chain or cyclic C₁₋₈ (per)fluoroalkyl group, an optionally substituted chain or cyclic C₁₋₈ (per) fluoroalkylene group. Even more preferably, X¹, X², X³, and X⁴ are the same or different and each represents a hydrogen atom, a fluorine atom, a chlorine atom, an optionally substituted oxygen atom, an optionally substituted chain or cyclic C₁₋₄ alkyl group, an optionally substituted C₁₋₄ alkylene group, a nitrile group, an optionally substituted C₁₋₈ fluorine-containing alkyl group, or an optionally substituted C₁₋₄ fluorine-containing alkylene group, and X and Y are the same or different and each represents a hydrogen atom, an optionally substituted chain or cyclic C₁₋₄ alkyl group, an optionally substituted chain or cyclic C₁₋₄ alkylene group, an optionally substituted chain or cyclic C₁₋₄ (per)fluoroalkyl group, an optionally substituted chain or cyclic C₁₋₄ (per)fluoroalkylene group, or an aryl group.

Examples of the polymer of the present disclosure include organopolysiloxanes such as polydimethylsiloxane and polydiethylsiloxane, polyacrylonitrile, polyvinyl chloride, polystyrene, polyethylene, partially chlorinated polyethylene, polypropylene, polyisoprene, polybutadiene, poly-2-chlorobutadiene, an ethylene-propylene copolymer, an ethylene-butylene copolymer, an ethylene-butadiene copolymer, a styrene-butadiene-styrene block copolymer, a styrene-isoprene-styrene block copolymer, a styrene-ethylene/butylene-butadiene block copolymer, a styrene-ethylene/propylene-butadiene block copolymer, an acrylonitrile-styrene-butadiene copolymer, and an ethylene-butylene block copolymer. The polymer of the present disclosure may entirely or partially have a cyclic structure.

The polymer of the present disclosure is preferably polydimethylsiloxane, polyacrylonitrile, polyvinyl chloride, polystyrene, poly-2-chlorobutadiene, a styrene-butadiene-styrene block copolymer, a styrene-isoprene-styrene block copolymer, a styrene-ethylene/butylene-butadiene block copolymer, a styrene-ethylene/propylene-butadiene block copolymer, an acrylonitrile-styrene-butadiene copolymer, an ethylene-butylene block copolymer, or the like. The polymer of the present disclosure may entirely or partially have a cyclic structure.

The structural unit constituting the main chain in the repeating unit in the main chain of the polymer of the present disclosure includes -CH₂-CH₂-, -CH₂-CHR-, -CH₂-CR₂-, -CH₂-CH(C(=O)OR)-, -CH₂-CH(OC(=O)R)-, -CH₂-CHOR-, -CH₂-CH(C₄H₆NO)-, -CH₂-CHCl-, -CH₂-CHF-, -CF₂-CF₂-, -CF₂-CFR-, -CF₂-CR₂-, -CF₂-CF(C(=O)OR)-, -CF₂-CF(OC(=O)R)-, -CF₂-CFOR-, -CF₂-CFCl-, -CHF-CF₂-, -CHF-CFR-, - CHF-CR₂-, -CHF-CF(C(=O)OR)-, -CHF-CF(OC(=O)R)-, -CHF-CFOR-, -CHF-CFCl-, -CF₂-CHR-, -CF₂-CH(C(=O)OR)-, -CF₂-CH(OC(=O)R)-, -CF₂-CHOR-, -CF₂-CHCl-, -CF₂-CHF-, -CH₂-CF₂-, -CH₂-CFR-, -CH₂-CR₂-, -CH₂-CF(C(=O)OR)-, -CH₂-CF(OC(=O)R)-, -CH₂-CFOR-, -CH₂-CFCl-, -CH=CH-, - CH=CF-, -CH=CR-, -CH₂-O-CH₂-, -CH₂-O-CHR-, -CH₂-O-CR²-, -CH₂-O-CH(C(=O)OR)-, -CH₂-O-CH(OC (=O) R) -, -CH₂-O-CHOR-, -CH₂-O-CH(C₄H₆NO)-, -CH₂-O-CHCl-, -CH₂-O-CHF-, -CF₂-O-CF₂-, -CF₂-O-CFR-, -CF₂-O-CR₂-, - CF₂-O-CF(C(=O)OR)-, -CF₂-O-CF(OC(=O)R)-, -CF₂-O-CFOR-, -CF₂-O-CFCl-, -CHF-O-CF₂-, -CHF-O-CFR-, -CHF-O-CR₂-, -CHF-O-CF(C(=O)OR)-, -CHF-O-CF(OC(=O)R)-, -CHF-O-CFOR-, -CHF-O-CFCl-, -CF₂-O-CHR-, -CF₂-O-CH(C(=O)OR)-, -CF₂-O-CH(OC(=O)R)-, -CF₂-O-CHOR-, -CF₂-O-CHCl-, -CF₂-O-CHF-, -CH₂-O-CF₂-, -CH₂-O-CFR-, -CH₂-O-CR₂-, -CH₂-O-CF(C(=O)OR)-, -CH₂-O-CF(OC (=O) R) -, -CH₂-O-CFOR-, -CH₂-O-CFCl-, -CH₂-C(=O)-CH₂-, - CH₂-C(=O) -CHR-, -CH₂-C(=O) -CR₂-, -CH₂-C(=O) -CH (C (=O) OR) -, -CH₂-C (=O) -CH (OC (=O) R) -, -CH₂-C(=O) -CHOR-, -CH₂-C(=O) -CH (C₄H₆NO)-, -CH₂-C (=O) -CHCl-, -CH₂-C(=O) -CHF-, -CF₂-C(=O)-CF₂-, -CF₂-C(=O) -CFR-, - CF₂-C(=O) -CR₂-, -CF₂-C(=O) -CF (C (=O) OR) -, -CF₂-C(=O) -CF (OC (=O) R) -, - CF₂-C(=O) -CFOR-, -CF₂-C(=O) -CFCl-, -CHF-C (=O) -CF₂-, -CHF-C (=O) -CFR-, -CHF-C (=O) -CR₂-, -CHF-C (=O) -CF (C (=O) OR) -, -CHF-C (=O) -CF (OC (=O) R) -, -CHF-C (=O) -CFOR-, -CHF-C (=O) -CFCl-, -CF₂-C(=O) -CHR-, -CF₂-C(=O) - CH(C(=O)OR)-, -CF₂-C(=O)-CH(OC(=O)R)-, -CF₂-C(=O) -CHOR-, -CF₂-C (=O) -CHCl-, -CF₂-C(=O) -CHF-, -CH₂-C(=O)-CF₂-, -CH₂-C(=O) -CFR-, - CH₂-C(=O)-CR₂-, -CH₂-C(=O) -CF (C (=O) OR) -, -CH₂-C(=O)-CF (OC (=O) R) -, - CH₂-C(=O)-CFOR-, -CH₂-C(=O)-CFCl-, -C(=O)-NR₂-, -SiR₂-SiR₂-, and - SiR₂-O-SiR₂- (wherein Rs are the same or different and each represents a chain or cyclic C₁₋₂₀ alkyl group optionally having at least one branch and/or functional group, an aryl group, a chain or cyclic alkyl group in which at least one hydrogen atom is replaced by a fluorine atom, an alkylene group in which at least one hydrogen atom is replaced by a fluorine atom, an aryl group in which at least one hydrogen atom is replaced by a fluorine atom, or a chain or cyclic perfluoroalkyl group).

The structural unit constituting the main chain in the repeating unit in the main chain of the polymer of the present disclosure is preferably -CH₂-CH₂-, -CH₂-CHR-, -CH₂-CR₂-, -CH₂-CH(C(=O)OR)-, -CH₂-CH(OC(=O)R)-, -CH₂-CHOR-, -CH₂-CH(C₄H₆NO)-, - CH₂-CHCl-, -CH₂-CHF-, -CF₂-CF₂-, -CF₂-CFR-, -CF₂-CR₂-, -CF₂-CF(C(=O)OR)-, -CF₂-CF(OC(=O)R)-, -CF₂-CFOR-, -CF₂-CFCl-, -CHF-CF₂-, -CHF-CFR-, -CHF-CR₂-, -CHF-CF(C(=O)OR)-, -CHF-CF(OC(=O)R)-, -CHF-CFOR-, -CHF-CFCl-, -CF₂-CHR-, -CF₂-CH(C(=O)OR)-, -CF₂-CH(OC(=O)R)-, -CF₂-CHOR-, -CF₂-CHCl-, -CF₂-CHF-, -CH₂-CF₂-, -CH₂-CFR-, -CH₂-CR₂-, -CH₂-CF(C(=O)OR)-, -CH₂-CF(OC(=O)R)-, -CH₂-CFOR-, -CH₂-CFCl-, -CH=CH-, -CH=CF-, -CH=CR-, -CH₂-O-CH₂-, -CH₂-O-CHR-, - CH₂-O-CR₂-, -CH₂-O-CH(C(=O)OR)-, -CH₂-O-CH(OC(=O)R) -, -CH₂-O-CHOR-, -CH₂-O-CH(C₄H₆NO)-, -CH₂-O-CHCl-, -CH₂-O-CHF-, -CF₂-O-CF₂-, -CF₂-O-CFR-, -CF₂-O-CR₂-, -CF₂-O-CF(C(=O)OR)-, -CF₂-O-CF(OC(=O)R)-, -CF₂-O-CFOR-, -CF₂-O-CFCl-, -CHF-O-CF₂-, -CHF-O-CFR-, -CHF-O-CR₂-, - CHF-O-CF(C(=O)OR)-, -CHF-O-CF(OC(=O)R)-, -CHF-O-CFOR-, -CHF-O-CFCl-, -CF₂-O-CHR-, -CF₂-O-CH(C(=O)OR)-, -CF₂-O-CH(OC(=O)R)-, -CF₂-O-CHOR-, -CF₂-O-CHCl-, -CF₂-O-CHF-, -CH₂-O-CF₂-, -CH₂-O-CFR-, -CH₂-O-CR₂-, -CH₂-O-CF(C(=O)OR)-, -CH₂-O-CF(OC(=O)R)-, -CH₂-O-CFOR-, - CH₂-O-CFCl-, -CH₂-C(=O)-CH₂-, -CH₂-C(=O)-CHR-, -CH₂-C(=O)-CR₂-, - CH₂-C(=O)-CH(C(=O)OR)-, -CH₂-C(=O)-CH(OC(=O)R)-, -CH₂-C(=O)-CHOR-, -CH₂-C(=O)-CH(C₄H₆NO)-, -CH₂-C(=O)-CHCl-, -CH₂-C(=O)-CHF-, -CF₂-C(=O)-CF₂-, -CF₂-C(=O)-CFR-, -CF₂-C(=O)-CR₂-, -CF₂-C(=O)-CF(C(=O)OR)-, -CF₂-C(=O)-CF(OC(=O)R)-, -CF₂-C(=O)-CFOR-, -CF₂-C(=O)-CFCl-, -CHF-C(=O)-CF₂-, -CHF-C(=O)-CFR-, -CHF-C(=O)-CR₂-, - CHF-C(=O)-CF(C(=O)OR)-, -CHF-C(=O)-CF(OC(=O)R)-, -CHF-C(=O)-CFOR-, -CHF-C(=O)-CFCl-, -CF₂-C(=O)-CHR-, -CF₂-C(=O)-CH(C(=O)OR)-, -CF₂-C(=O)-CH(OC(=O)R)-, -CF₂-C(=O)-CHOR-, -CF₂-C(=O)-CHCl-, -CF₂-C(=O)-CHF-, -CH₂-C(=O)-CF₂-, -CH₂-C(=O)-CFR-, -CH₂-C(=O)-CR₂-, - CH₂-C(=O)-CF(C(=O)OR)-, -CH₂-C(=O)-CF(OC(=O)R)-, -CH₂-C(=O)-CFOR-, -CH₂-C(=O)-CFCl-, -C(=O)-NR₂-, -SiR₂-SiR₂-, and -SiR₂-O-SiR₂-(wherein Rs are the same or different and each represents a chain or cyclic C₁₋₁₀ alkyl group optionally having at least one branch and/or functional group, an aryl group, a chain or cyclic alkyl group in which at least one hydrogen atom is replaced by a fluorine atom, an aryl group in which at least one hydrogen atom is replaced by a fluorine atom, or a chain (per)fluoroalkyl group).

It is advantageous that the polymer of the present disclosure can be dissolved in a solvent during the microparticle production process. The solvent that can be used in the production process is not limited, and is preferably an aprotic polar solvent, a nonpolar solvent, a low-polar solvent, an alcohol solvent, a fluorine solvent, and the like. Examples of the aprotic polar solvent include N,N-dimethylformamide, acetonitrile, dimethyl sulfoxide, hexamethylphosphate triamide, N-methylpyrrolidone, ethyl acetate, dimethyl carbonate, acetone, methyl ethyl ketone, tetrahydrofuran, and dioxane. Examples of the nonpolar solvent include carbon tetrachloride, benzene, toluene, hexane, and cyclohexane. Examples of the low polar solvent include diethyl ether, monogram, dichloromethane, and chloroform. Examples of the alcohol solvent include methanol, ethanol, 1-propanol, 2-propanol, ethylene glycol, glycerin, and mannitol. Examples of the fluorine-based solvent include perfluorohexane, perfluorobenzene, hydrofluoroether, perfluoro crown ether, 1-bromoheptadecafluorooctane, 1-bromotridecafluorohexane, perfluorodecalin, hexafluoroisopropanol, and 2-perfluorohexyl ethanol. These solvents may be used alone or in a combination of two or more. When multiple solvents are combined, they are preferably compatible, and examples include a combination of ethyl acetate and hexane, ethyl acetate and methanol, acetone and tetrahydrofuran, hydrofluoroether and dichloromethane, hydrofluoroether and acetone, or the like.

The polymer of the present disclosure preferably exhibits solubility in solvents used in these production processes. The solubility in these solvents is preferably 1 mg/L or more, more preferably 100 mg/L or more, and even more preferably 1000 mg/L or more.

The polymer of the present disclosure may be obtained by polymerizing a single monomer or obtained by copolymerizing multiple types of monomers.

It is preferable that the polymer of the present disclosure exhibits a small change in physical properties even after the application of acidic or alkaline conditions (for example, immersion in a 10% hydrochloric acid or 20% sodium hydroxide aqueous solution for one week at room temperature). It is preferable that the change in physical properties is small even after the application of hot-water conditions (e.g., 50 to 100°C).

Examples of the change in physical properties include tensile strength and tensile elongation.

The polymer of the present disclosure may be a fluorine-containing polymer. In this case, due to the low intermolecular force and low surface free energy of the fluorine-containing polymer, the microparticle of the present disclosure can be dispersed in an aqueous medium. Since the shell contains a fluorine-containing polymer, the core containing the fluorocarbon therein can be stably stored. In addition, the fluorine-containing polymer has excellent stability to acid, alkali, or hot water, and also has excellent long-term stability in water. In addition, there is an advantage such that the surface is smooth and the growth of microorganisms is small. Therefore, the type of the fluorine-containing polymer is not limited.

The fluorine-containing polymer may be obtained by polymerizing a single monomer or by copolymerizing multiple monomers.

When the fluorine-containing polymer is a homopolymer, it may be, for example, a polymer obtained by polymerizing a fluoroacrylic monomer. Examples of such monomers include methyl α-fluoroacrylate, ethyl α-fluoroacrylate, trifluoroethyl α-fluoroacrylate, pentafluoropropyl α-fluoroacrylate, isopropyl α-fluoroacrylate, hexafluoroisopropyl α-fluoroacrylate, cyclohexyl α-fluoroacrylate, phenyl fluoroacrylate, 2,2,2-trifluoroethyl (meth)acrylate, 2,2,3,3-tetrafluoropropyl (meth)acrylate, 1,1,1,3,3,3-hexafluoroisopropyl(meth)acrylate, heptafluoroisopropyl(meth)acrylate, 1,1-dihydroheptafluoro-n-butyl(meth)acrylate, 1,1,5-trihydrooctafluoro-n-pentyl(meth)acrylate, 1,1,2,2-tetrahydrotridecafluoro-n-octyl (meth)acrylate, and 1,1,2,2-tetrahydroheptadecafluoro-n-decyl (meth)acrylate.

When the fluorine-containing polymer is a homopolymer, other examples include (per)fluoro-1,3-dioxol, (per)fluorobutenyl vinyl ether, (per)fluoro 2,2-dimethyl-1,3-dioxol, and (per)fluoro-2-methylene-4-methyl-1,3-dioxolane. In the present disclosure, "perfluoro" means that all of the hydrogen atoms in the hydrocarbon are replaced by fluorine atoms, and "(per)fluoro" indicates either "perfluoro" or "fluoro."

When the fluorine-containing polymer is a copolymer, it may be a copolymer of a fluorine-containing monomer and a fluorine-free monomer, or may be a copolymer of fluorine-containing monomers. In this case, either or both of the fluorine-containing monomer and the fluorine-free monomer forming the copolymer may be two or more kinds of monomers. Examples of the fluorine-containing monomer include vinyl fluoride monomer, (per)fluorobutenyl vinyl ether, (per)fluoro-2,2-dimethyl-1,3-dioxol, (per)fluoro-2-methylene-4 methyl-1, 3-dioxolane, and (per)fluoro-1,3-dioxol. Examples of the vinyl fluoride monomer include vinylidene fluoride, tetrafluoroethylene, and hexafluoropropylene, and those represented by the following formula: 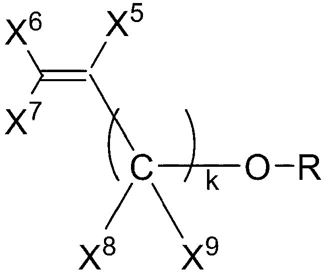 wherein k represents 0 or 1, and X⁵ to X⁹ are the same or different and each represents a hydrogen atom or a fluorine atom, R represents a chain or cyclic C₁₋₁₀ alkyl group optionally having at least one branch and/or functional group, an aryl group, an alkyl group in which at least one hydrogen atom is replaced by a fluorine atom, an aryl group in which at least one hydrogen atom is replaced by a fluorine atom, or a chain or cyclic perfluoroalkyl group.

Examples of the fluorine-free monomer include a vinyl monomer and a cyclic monomer. Examples of the vinyl monomer include acrylonitrile, methyl acrylate, methyl methacrylate, vinyl pyrrolidone, isoprene, 1,3-butadiene, styrene, vinyl chloride, vinyl acetate, ethylene, propylene, and isobutene. Examples of the cyclic monomer include those having a structure of ether, sulfide, carbonate ester, phosphate ester, lactone, lactam, and siloxane.

Examples of other fluorine-containing polymers include the following. Examples of the fluorine-containing polymer (a solvent-soluble fluorine-containing polymer mainly used as a material for melt molding) include thermoplastic fluororesins such as an ethylene/tetrafluoroethylene copolymer (ETFE), a vinylidene fluoride polymer (PVDF), a tetrafluoroethylene/vinylidene fluoride copolymer (monomer molar ratio = 50/50 to 99/1), a tetrafluoroethylene/vinylidene fluoride/third monomer copolymer (the third monomer may be a fluorine-containing monomer or a fluorine-free monomer, and accounts for 10 mol% or less of all monomer units), a tetrafluoroethylene/hexafluoropropylene/vinylidene fluoride copolymer (THV), an ethylene/tetrafluoroethylene/hexafluoropropylene copolymer (EFEP), a chlorotrifluoroethylene polymer (PCTFE), a tetrafluoroethylene/ion-exchange-functional-group (carboxylic acid group, sulfonic acid group, sulfonimide group, etc.)-containing perfluoroalkyl vinyl ether copolymer, or a precursor thereof.

Examples of the fluorine-containing polymer include a copolymer of tetrafluoroethylene/non-aromatic vinyl ester monomer free of a hydroxyl group and a carboxyl group/hydroxyl group-containing vinyl monomer free of an aromatic group and a carboxyl group (monomer molar ratio = 20 to 49/25 to 69.9/8 to 30) (see WO2003/106516), and a copolymer of chlorotrifluoroethylene/cyclohexyl vinyl ether/alkyl vinyl ether/hydroxyalkyl vinyl ether(monomer molar ratio=40 to 60/5 to 45/5 to 45/3 to 15) (see Japanese Unexamined Patent Application Publication No. S57-34107 etc.).

Examples of the fluorine-containing polymer include a thermoplastic fluororesin such as a copolymer containing a fluoroolefin unit and a polymerizable vinyl compound unit having an amide group (monomer molar ratio = 7 to 65/35 to 93) (see WO2016/133206).

The monomer molar ratio of the fluoroolefin repeating unit and a hydroxyl group-containing repeating unit in the copolymer having the fluoroolefin repeating unit and a hydroxyl group-containing repeating unit is preferably 10 to 60/40 to 90.

The fluorine-containing polymer also includes fluororubber. Examples of the fluororubber include a thermoplastic elastomer of a tetrafluoroethylene/propylene copolymer, a tetrafluoroethylene/propylene/third monomer copolymer (the third monomer being vinylidene fluoride, hexafluoropropylene, chlorotrifluoroethylene, fluoroalkyl vinyl ether, or the like), hexafluoropropylene/ethylene copolymer, hexafluoropropylene/ethylene/tetrafluoroethylene copolymer, vinylidene fluoride/hexafluoropropylene copolymer, vinylidene fluoride/tetrafluoroethylene/hexafluoropropylene copolymer, and vinylidene fluoride/2,3,3,3-tetrafluoropropylene copolymer.

The fluorine-containing polymer also includes an amorphous fluororesin. The amorphous fluororesin is a copolymer containing the constituent unit represented by the following formula (A1), 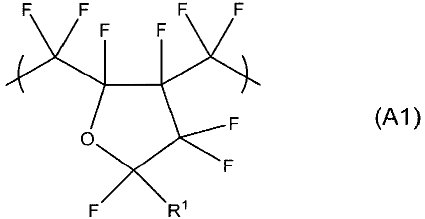 wherein R¹ represents a fluorine atom or a C₁₋₅ (per) fluoroalkyl group. Examples include a copolymer containing the structural units represented by the following formulas (A2) and (A3): 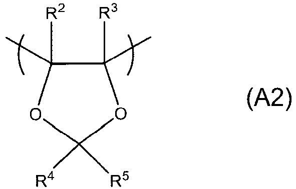 wherein R² to R⁵ each independently represent a fluorine atom, a C₁-₅ (per)fluoroalkyl group, or a C₁-₅ (per)fluoroalkoxy group, and 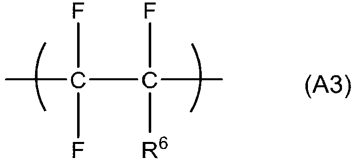 wherein R⁶ represents a fluorine atom, a C₁-₆ (per) fluoroalkyl group, or a C₁-₆ (per)fluoroalkoxy group; and a copolymer comprising a structural unit represented by the following formula (A4): 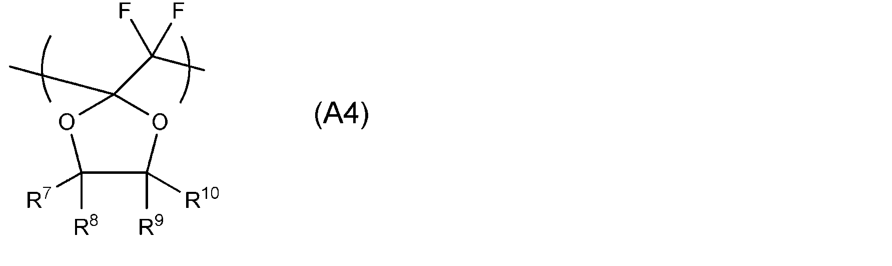 wherein R⁷ to R¹⁰ independently represent a fluorine atom, a C₁₋₅ (per)fluoroalkyl group, or a C₁-₅ (per)fluoroalkoxy group.

Examples of the fluorine-containing polymer having an epoxy structure include a compound (B) represented by the following formula (B): 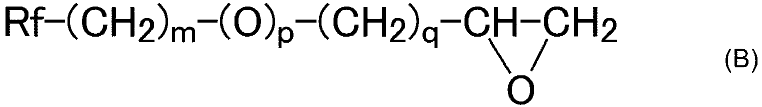
wherein m is an integer of 0 to 6, p is 0 or 1, and q is an integer of 0 to 6, Rf is a C₁₋₈ (per)fluoroalkyl group optionally containing an oxygen, wherein a fluorine atom may be substituted with a hydrogen atom);
a compound (C) represented by the following formula (C): 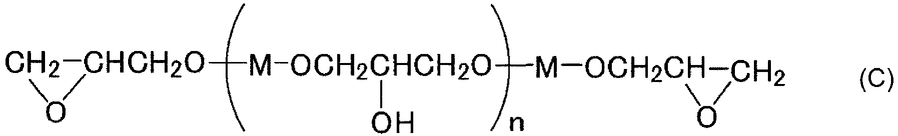
wherein n is an integer of 0 or more, M is a group represented by the following formula (C1): 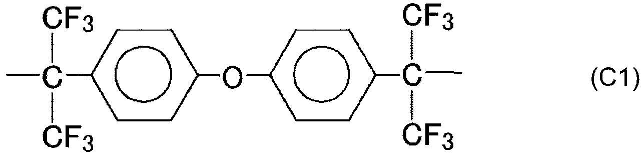
a group represented by the following formula (C2): 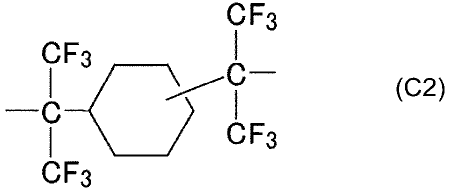
or a group represented by the following formula (C3): 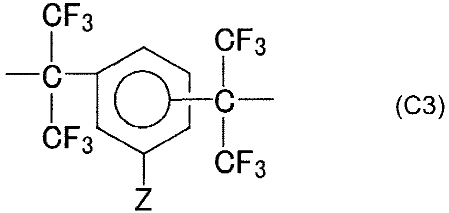
wherein Z is hydrogen or a C₁₋₁₀ (per) fluoroalkyl group.

In formula (C), M is preferably a group represented by the following formula (C4):

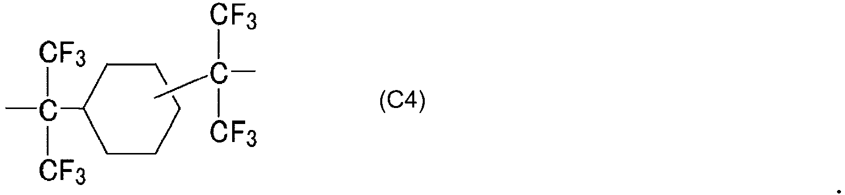

In formula (B), the Rf is preferably a linear C₆ (per)fluoroalkyl group.

The compound (B) and the compound (C) can be used as a composition containing a respective compound, and the compound (B) is preferably 5 to 95 mass% based on 100 mass% in total. Such compositions can be used as curable compositions in combination with a curing agent. The curing agent is preferably at least one member selected from the group consisting of an acid anhydride, an amine, an isocyanate, an amide, an imidazole, and a mercaptan. Further, these compositions may include a non-fluorine-containing epoxy resin.

The compound (B) also provides a composition comprising a compatibilizer and having a fluorine content of 20 mass% or more. The fluorine content is preferably 30 mass% or more, more preferably 40 mass% or more, and still more preferably 45 mass% or more.

The compatibilizer is preferably a fluorine-containing compound containing two or more epoxy groups and an aromatic ring or a cyclohexane ring in the molecular structure.

As a specific compound of the compound (B), a compound represented by the following chemical formula is preferable. These compounds may be used alone or in a combination of two or more.

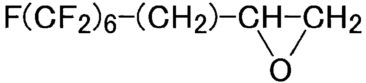

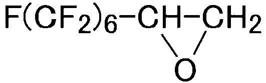

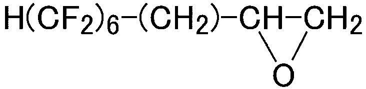

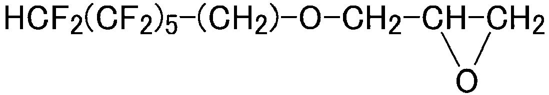

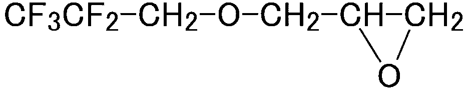

As the fluorine-containing polymer, a fluorine-containing polyimide containing the following structure as a repeating unit can be used. 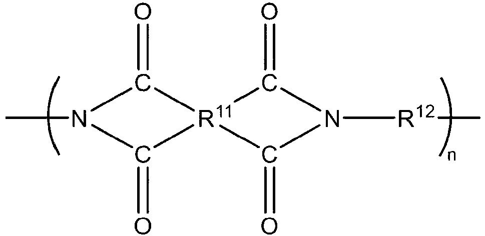 In the formula, R¹¹ represents an aromatic ring structure, and R¹¹ forms rings of 5 or 6 atoms with a respective adjacent imide group.
R¹¹ is preferably a group represented by the following formula: 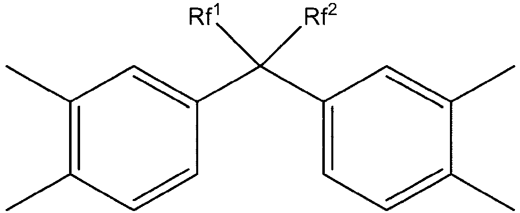
wherein Rf¹ and Rf² are the same or different and each represents a C₁₋₁₀ perfluoroalkyl group.
   R¹² represents a carbon chain portion of a diamine represented by NH₂-R¹²-NH₂, and n is an integer of 1 or more.

R¹² is not limited, and is a C₁₋₃₀ fluorine-containing alkyl chain optionally having a branch and an ether functional group in the main chain. The internal structure of R¹² may include multiple repeating units.

The fluorine-containing polymer is not limited as long as it has solvent solubility; however, a fluorine-containing silicone having the following structure can be used. 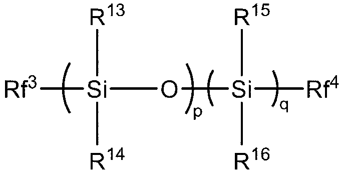 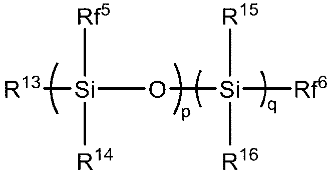 In the formula, Rf³ to Rf⁶ are the same or different and each represents a C₁₋₂₀ perfluoroalkyl group, and R¹³ to R¹⁶ are the same or different and each represents a C₁₋₁₀ alkyl group or an aryl group. In addition, Rf³ to Rf⁶ and R¹³ to R¹⁶ optionally have at least one branch or at least one member selected from an oxygen atom, a sulfur atom, an NH group, and a hydroxyl group, and p and q are each an integer within the range of 0 to 1000 and they cannot be simultaneously 0.)

The fluorine-free vinyl polymer may be obtained by polymerizing a single vinyl monomer or copolymerizing multiple vinyl monomers.

When the fluorine-free vinyl polymer is a homopolymer, it may be, for example, a polymer obtained by polymerizing ethylene, propylene, vinyl chloride, vinyl acetate, acrylonitrile, (meth)acrylic acid ester, styrene, butadiene, and isoprene. Examples of the (meth)acrylic acid ester include methyl(meth)acrylate, ethyl(meth)acrylate, trifluoroethyl(meth)acrylate, pentafluoropropyl(meth)acrylate, isopropyl(meth)acrylate, hexafluoroisopropyl(meth)acrylate, cyclohexyl(meth)acrylate, phenyl(meth)acrylate, and stearyl(meth)acrylate.

The silicone polymer is not limited as long as it has solvent solubility, and the silicone polymer having the following structure can be used. 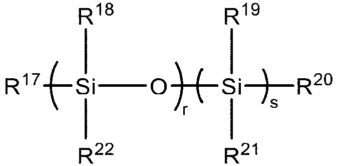 In the formula, R¹⁷ to R²² optionally have at least one branch or at least one member selected from an oxygen atom, a sulfur atom, an NH group, and a hydroxyl group, and r and s are each an integer within the range of 0 to 10000 and they cannot be simultaneously 0.

The shell may contain two or more different types of polymers, and examples of the combination of polymers include a combination of fluorine-containing polymers, a combination of a fluorine-containing polymer and a fluorine-free vinyl polymer, and a combination of a fluorine-containing polymer and a silicone polymer. Additionally, it is more preferable that the compatibility of a group of composite polymers, such as fluorine-containing polymers, is high.

In addition to the polymer of the present disclosure, the shell may further comprise other components. Examples of such components include at least one member selected from the group consisting of imaging dyes, drugs, and radical scavengers such as vitamin C and vitamin E. The radical scavenger can be added for the purpose of suppressing side effects when toxicity may occur due to oxygen exposure.

When the shell contains a fluorine-containing polymer, the fluorine content of the shell is preferably 10% or more, more preferably 20 mass% or more, still more preferably 30 mass% or more, even more preferably 40 mass% or more, and particularly preferably 45 mass% or more.

It is preferable that the shell has deformability because the microparticle easily passes through a narrow tube having a size about 1 to 50 µm, such as a capillary or a microchannel. In this respect, the elastic modulus of the shell is preferably 500 MPa or less, more preferably 300 MPa or less, still more preferably 100 MPa or less, and particularly preferably 50 MPa or less. Typically, the elastic modulus of the shell is preferably 0.1 MPa or more, more preferably 0.5 MPa or more, and even more preferably 1 MPa or more from the viewpoint of moldability, shape retention, and the like.

The elastic modulus of the shell can be measured by the following tensile test.

### (1) Preparation of Test Films

The test piece for the tensile test is obtained by dissolving the shell in a solvent such as chloroform, coating the shell on a glass substrate, followed by drying to form a film of about 200 µm.

### (2) Tensile Test

The tensile test is carried out with a tensile tester (CR-3000 EX-S, produced by Sun Science Co., Ltd.) at a displacement rate of 5 mm/min. The measurement is performed at room temperature and 50 ± 5% relative humidity (RH) according to ISO 527-3/1995 (ISO 527-3/2/5) after the formation of a test piece and storage at room temperature (21±2°C) for 5 weeks. The elastic modulus of the test piece is obtained from the tensile test result.

### 1.2 Core

The microparticle of the present disclosure comprises a core, the core comprising a fluorocarbon. The fluorocarbon has excellent oxygen solubility. The microparticle of the present disclosure can be preferably used as an oxygen carrier.

In the present disclosure, "fluorocarbon" means an organic fluorine compound having a carbon-fluorine bond. Fluorocarbons are capable of reversibly occluding and/or adsorbing a gas, such as oxygen, carbon dioxide, nitrogen, and hydrogen. Due to the reversible nature of their ability to occlude gases, fluorocarbons can release a gas under physiological conditions (in particular, under an environment in which the amount of gas, such as oxygen, is insufficient). For example, fluorocarbons can release a required gas, such as oxygen, to ischemic tissues and/or organs. Further, fluorocarbons have excellent stability and sterility.

Examples of the gas occluded in and/or adsorbed (dissolved) on the fluorocarbon include, but are not limited to, oxygen, carbon dioxide, and nitrogen. When the gas is oxygen, the microparticle of the present disclosure can be used as an artificial oxygen carrier.

The fluorocarbon to be used is in liquid or gel form, and preferably in liquid form. It is particularly preferable that a liquid under ambient or physiological conditions. In the present disclosure, "physiological conditions" refer to conditions that include at least *in vivo* chemical conditions (temperature, pressure, pH). For example, the fluorocarbon is preferably a liquid at atmospheric pressure and room temperature (e.g., 10 to 35°C).

The type of fluorocarbon is not limited but is preferably a fluorocarbon that has excellent gas occlusion capacity (gas solubility). For example, liquid fluorocarbons having an oxygen solubility of 30 vol/vol% or more (preferably 40 vol/vol% or more, and more preferably 50 vol/vol% or more, and still more preferably 60 vol/vol% or more) are preferred. In the present disclosure, "oxygen solubility" refers to the solubility of an oxygen molecule in a fluorocarbon at 25°C.

The weight average molecular weight of the fluorocarbon is preferably in the range of 150 to 50000, more preferably 250 to 45000, and even more preferably 350 to 40000 from the viewpoint of gas solubility. The weight average molecular weight of the fluorocarbon is preferably 5000 or more, more preferably 7500 or more, and even more preferably 10000 or more. The weight average molecular weight of the fluorocarbon is preferably as high as possible, as long as sufficient solubility in a solvent can be ensured.

The molecular weights in the present disclosure all refer to weight average molecular weight. The molecular weights mean values measured by GPC: gel permeation chromatography.

The fluorocarbon is not limited as long as it can reversibly occlude and/or adsorb a gas. Fluorocarbons known so far as oxygen carriers or oxygen carrier candidates can also be used in the present disclosure. Examples of such fluorocarbons include at least one member selected from the group consisting of hydrofluoroethers, fluoroalkanes, bis(fluoroalkyl)ethenes, cyclic (per)fluorocarbons, (per)fluoroamines, (per)fluoro(2-butyltetrahydrofuran), (per)fluorocarbon bromides, (per)fluorocarbon iodides, (per)fluorocarbon chlorides, (per)fluoroalkyl ethers, (per)fluoroalkyl polyethers, and (per)fluoropolyethers that may contain one or more functional groups other than fluorine at the ends. In the present disclosure, "perfluoro" means that all hydrogen atoms of a hydrocarbon are replaced by fluorine atoms, and "(per)fluoro" means either "perfluoro" or "fluoro."

Examples of hydrofluoroethers include methyl-(per)fluorobutyl ether, ethyl-(per)fluorobutyl ether, methyl perfluorohexyl ether, and hexafluoropropyl (methyl)methyl-hexafluoropropyl ether.

Examples of fluoroalkanes include (per)fluorohexane, (per)fluorocyclohexane, (per)fluoromethylcyclohexane, (per)fluorooctane, perfluorooctyldecane, perfluorohexyldecane, and perfluorooctyldodecane.

Examples of bis(fluoroalkyl)ethenes include C₄F₉CH=CH₄CF₉, i-C₃F₉CH=CHC₆F₁₃, and C₆F₁₃CH=CHC₆F₁₃.

Examples of cyclic (per)fluorocarbons include (per)fluorodecalin, (per)fluoroadamantane, (per)fluoromethyladamantane, F-1,3-dimethyladamantane, (per)fluorodimethylbicyclo[3,3,1]nonane, (per)fluorotrimethylbicyclo[3,3,1]nonane, (per)fluorocyclohexylmethylmorpholine, (per)fluorocyclohexyloxyethylmorpholine, (per)fluorocyclohexylmethylhexamethyleneimine, (per)fluorocycloheptylmorpholine, (per)fluoro-2,6-dimethyl-4-cyclohexylmorpholine, (per)fluoroethoxyethylpiperidine, (per)fluoro-2-ethoxydecalin, (per)fluoromethylcyclohexane, (per)fluoropropylcyclohexane, (per)fluoro-1,4-dioxabicyclo-4,4,0-decane, (per)fluoromethyl-dioxabicyclo-4,4,0-decane, (per)fluoroethoxymethyldioxabicyclo-4,4,0-decane, 1-methyl-1,2,2,3,3,4,4,4,5,5-nonafluorocyclopentane, 1-fluoromethyl-1,2,2,3,3,4,4,5,5-nonafluorocyclopentane, and 1-ethyl-1,2,2,3,3,4,4,5,5-nonafluorocyclopentane.

Examples of (per)fluoramines include (per)fluorotripropylamine, (per)fluorotributylamine, F-4-methyloctahydroquinolizine, F-n-methyl-decahydroisoquinoline, F-n-methyl-decahydroquinoline, F-n-cyclohexylpyrrolidine, F-n-(4-methylcyclohexyl)piperidine, (per)fluorodibutylmethylamine, 1H,1H-undecafluorohexylamine, and 1H,1H-(per)fluoroheptylamine.

Examples of (per)fluorocarbon bromides include (per)fluorooctyl bromide, (per)fluorodecyl bromide, (per)fluorohexyl bromide, 1-bromopentadecafluoroseptane, and 1-bromo-1H,1H,2H,2H-(per)fluorodecane.

Examples of (per)fluorocarbons include (per)fluorooctyl iodide and 1-iodo-8H-(per)fluorooctane.

Examples of (per)fluorocarbon chlorides include 1,8-perfluorodichlorooctane.

The (per)fluorocarbon to be used is preferably one that is a liquid at room temperature and that has a boiling point of 90°C or higher. The boiling point is more preferably 110°C or higher, and still more preferably 130°C or higher.

Examples of usable (per)fluoroalkyl ethers or (per)fluoroalkyl polyethers include compounds that are represented by the following formula and that are liquids at room temperature.

Rf⁷-O-(Rf^{x}O)ₘ-Rf⁸

(wherein Rf⁷ and Rf⁸ represent a C₁-C₁₆ (per) fluoroalkyl group optionally substituted with one or more fluorine atoms; Rf^{x} is a (per)fluoroalkylene group; and m is 1 to 300, and more preferably 20 to 300, with the proviso that (Rf^{x}O)ₘ may be composed of two or more compounds represented by Rf^{x}O that are different in number of carbon atoms) .

Specific examples of Rf⁷ and Rf⁸ include (CF₃)₂CF, CF₃CF₂CF₂, CF₃CF₂, CF₃, CF₂H, CFH₂, CH₂FCF₂CF₂, and CHF₂CF₂CF₂, and Rf⁷ and Rf⁸ may be the same or different.

Specific examples of Rf^{x} include CH₂CH₂CF₂CF₂, CH₂CF₂CF₂CF₂, CF₂CF₂CF₂CF₂, CH₂CH₂CH₂CF₂, CH₂CF₂CF₂CH₂, CH₂CF₂CF₂, CHFCF₂CF₂, CF₂, CFH, CF₂CF₂, CFHCF₂, CFHCFH, CF(CF₃)CF₂, and CF₂CF(CF₃), preferably CH₂CF₂CF₂, CF₂, CFH, CF₂CF₂, and CF(CF₃)CF₂.

Specific examples of (per)fluoroalkyl ethers or (per) fluoroalkyl polyethers include (CF₃)₂CFO(CF₂CF₂)₂OCF(CF₂H)₂, (CF₃)₂CFO(CF₂CF₂)₃OCF(CF₂H)₂, (CF₃)CFO(CF₂CF₂)H), (CF₃)₂CFO(CF₂CF₂)₂H, (CF₃)₂CFO(CF₂O)₂(CF₂CF₂)₂OCF(CF₂H)₂, (CF₃)₂CFO(CF₂CF₂)₃OCF(CF₂H)₂, (CF₃)CFO(CF₂O)₅(CF₂CF₂)H), (CF₃)₂CFO(CF₂CF₂)₂H, (CH₃)₂CFO(CF₂CF₂)₂H, and other esters represented by the following formula: CF₃CF₂CF₂O(CF₂CF₂O)ₜCF₂CF₃, CF₃CF₂CF₂O(CF₂CF₂CF₂O)ᵤCF₂CF₃, CF₃O(CF₂CF₂O)ᵥ(CF₂O)_{w}CF₃, and CF₃O(CF(CF₃)CF₂O)_{α}CF₂CF₃ (wherein t, u, v, w, and α each represent 0 to 300 in a range that they are not 0 at the same time, and the compounds may be used singly, in combination, or as a mixture of different repeating units).

The (per)fluoroalkyl ether or (per)fluoroalkyl polyether to be used is preferably one that is a liquid at room temperature and that has a boiling point of 130°C or higher. The boiling point is more preferably 150°C or higher, and still more preferably 200°C or higher.

The viscosity of the (per)fluoroalkyl ether or (per)fluoroalkyl polyether is preferably in the range of 10 to 3000 cSt (centistokes), more preferably 30 to 2000 cSt, as the kinematic viscosity at 20°C. In the present disclosure, the viscosity can be measured using a commercially available viscometer.

The fluorocarbons may be used singly or as a mixture of two or more.

Mixed fluorocarbon-hydrocarbon compounds modified into esters, thioethers, or various other forms are also included within the scope of "fluorocarbon" materials suitable for use in the present disclosure, in a broad sense. Other "fluorocarbons" not listed herein but having the properties described herein can also be used. Further, gel-like fluorocarbons to which a gelling agent has been added can also be used.

In view of gas occlusion capacity, the fluorocarbon content (mass ratio) in the microparticle is preferably 30 mass% or more, more preferably 40 mass% or more, and still more preferably 60 mass% or more, based on the mass of the microparticle (100 mass%). On the other hand, the fluorocarbon content is, for example, 95 mass% or less, or 90 mass% or less, based on the mass of the microparticle (100 mass%). For example, the fluorocarbon content is in the range of 30 to 95 mass% or in the range of 30 to 90 mass%. The fluorocarbon content (mass ratio) is a ratio of the mass of fluorocarbon to the total mass of the shell and the core (i.e., not including the gas dissolved in the fluorocarbon).

The mass ratio of the core component and the shell component that constitute the microparticle (core/shell) is, for example, in the range of 30/70 to 95/5, preferably 30/70 to 90/10, more preferably 40/60 to 90/10, and still more preferably 60/40 to 90/10.

### 2. Method for producing microparticle

Although the production method is not limited, the microparticle of the present disclosure can be produced, for example, by the following membrane emulsification method via a porous membrane.

The membrane emulsification method is advantageous in that (1) microparticles having a uniform particle size distribution (monodisperse, low CV value) can be obtained and that (2) the particle size (particle diameter) of the microparticles and the thickness of the shell can be controlled. Specifically, the membrane emulsification method comprises the following steps:
Step 1: preparing a dispersed phase by dispersing the fluorocarbon of the present disclosure and the polymer of the present disclosure in an organic solvent as described in the above 1 and subjecting the dispersed phase to membrane emulsification in a continuous phase containing a surfactant via a porous membrane having a uniform pore diameter to obtain an emulsion; and
step 2: removing the organic solvent phase contained in the emulsion to thereby form a core-shell microparticle composed of a shell comprising the polymer and a core comprising the fluorocarbon.

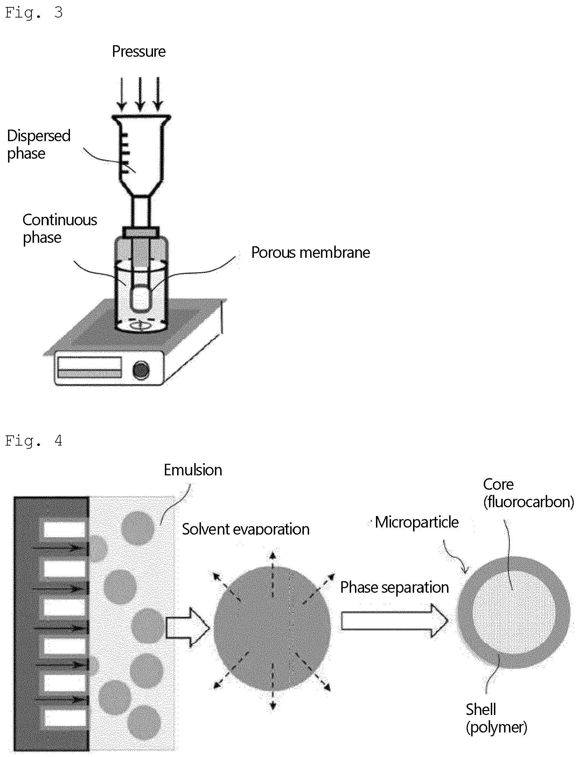

Each step is described below with reference to Figs. 3 and 4. Fig. 3 is a schematic diagram showing a manufacturing apparatus used in a method for producing the microparticle according to one embodiment of the present disclosure. Fig. 4 is a diagram illustrating the principle of a membrane emulsification method via a porous membrane.

In step 1, first, a dispersion in which a core component comprising the fluorocarbon and a shell component comprising the polymer are dispersed in an organic solvent is prepared as a dispersed phase. The organic solvent is preferably a low-boiling-point solvent that is capable of dissolving both the core component comprising the fluorocarbon and the shell component comprising the polymer, and that is immiscible with water. The organic solvent can be appropriately selected according to the type of polymer of the shell component. Specifically, solvents that are poorly miscible with water, such as dichloromethane, chloroform, and fluorine-based solvents, are preferred. The thickness of the shell of the microparticle as the final product can be controlled by adjusting the ratio of the core component, such as a fluorocarbon contained in the dispersion, to the shell component comprising a polymer.

Subsequently, as shown in Fig. 3, the dispersion prepared as a dispersed phase is pressed into a continuous phase containing a surfactant via a porous membrane by applying pressure using, for example, nitrogen gas, and is subjected to membrane emulsification. Specifically, as shown in Fig. 4, a dispersed phase, which is an oil (O) phase, is extruded into a continuous phase, which is a water (W) phase, through a porous membrane to form an O/W emulsion. The size of the emulsion formed is uniform (monodisperse) in correlation with the uniform pore diameter of the porous membrane. Further, since the size of the emulsion formed is proportional to the pore diameter of the porous membrane, the size of the emulsion formed and the size (particle diameter) of the microparticle can be controlled by adjusting the size of the pore diameter of the porous membrane. The volume ratio of the dispersed phase to the continuous phase (dispersed phase/continuous phase) is preferably maintained in the range of 1/2 to 1/1000 (more preferably 1/5 to 1/100, and particularly preferably about 1/10).

The porous membrane is not particularly limited as long as the porous membrane has a uniform pore diameter. The porous membrane can be made of glass, a resin such as polycarbonate, a metal, or like various materials. The pore diameter of the porous membrane may be determined according to the desired size of the microparticle. A porous membrane having a pore diameter in the range of 0.05 to 20 µm (preferably 2 to 10 µm) can be usually used.

The membrane emulsification is preferably performed with stirring in order to separate droplets from the membrane surface.

For example, an aqueous solution containing a surfactant can be used as the continuous phase.

The surfactant is not limited, and known surfactants used for forming an emulsion can be preferably used. Examples of usable surfactants include polyvinyl alcohol; and pluronic (registered trademark) surfactants, which are block copolymers of polyethylene oxide and polypropylene oxide.

Examples of the aqueous solvent include water, phosphate-buffered saline, culture media, and physiological saline. The type and concentration of surfactant used affects the size of the emulsion. Specifically, when the surfactant is used in a low concentration, the size of the emulsion formed tends to be large. The concentration of the surfactant in the aqueous solution is preferably 0.01 to 10 wt.%, and more preferably 0.1 to 3 wt.%, based on the total weight (100 wt.%) of the aqueous solution (aqueous solvent and surfactant).

In step 2, the organic solvent phase in the emulsion obtained in step 1 is removed by evaporation or the like. In the process of evaporation, phase separation occurs between the shell component comprising a polymer (O phase), the core component comprising a fluorocarbon (F phase), and the continuous phase (W phase), thus forming a core-shell type particle composed of the shell comprising a polymer and the core comprising a fluorocarbon. After the particle is formed, the particle is washed with, for example, pure water, to remove the surfactant.

In any case, depending on the application, the particle can be used as an emulsified particle after performing step 1 or used as a dried core-shell particle after performing step 2. The method for producing the microparticle of the invention is as disclosed in the appended claims.

### 3. Method for producing microparticle having a dent

The microparticle having a dent according to the present invention can be produced by a method comprising the following steps:
step 1: the step of adding an alcohol to a dispersion obtained by dispersing the microparticle of the invention in an aqueous medium, and
step 2: the step of resuspending the particle obtained in step (1) in a solvent.

First, in step 1, a dispersion in which a microparticle is dispersed in an aqueous medium (a first dispersion) is prepared, and an alcohol is added to the dispersion to thereby obtain a dispersion in which the microparticle is dispersed in a mixture of the alcohol and the aqueous medium (a second dispersion).

Examples of the aqueous medium include, but are not limited to, water, a mixed solution of water and an alcohol (for example, a lower alcohol), PBS (phosphate-buffered saline), culture media, and physiological saline. The type of alcohol to be added is not limited, and is preferably a lower alcohol (e.g., C₁ to C₆ alcohols and C₁ to C₃ alcohols). Among them, for example, isopropanol, ethanol, and methanol are preferable in terms of compatibility with water.

The concentration of the microparticle in the dispersion is not limited. For example, the dispersion preferably contains the microparticle in a concentration of 0.1 to 20 wt.%, based on the total weight of the dispersion (aqueous medium and the microparticle).

A larger amount of alcohol added tends to increase the yield of the microparticle having a dent. On the other hand, when the alcohol is added in an excess amount, the particles tend to aggregate. From this viewpoint, the amount of alcohol added is preferably 1 to 60 parts by volume, and more preferably 10 to 50 parts by volume, based on the amount of water in the aqueous medium (100 parts by volume).

After adding the alcohol, the second dispersion is preferably allowed to stand with stirring (preferably at a slow rate at which the particles do not settle) for 6 to 48 hours (more preferably for 8 to 36 hours, and still more preferably for 10 to 24 hours). The temperature during the standing is not limited, but is usually room temperature (for example, 10 to 35°C).

Subsequently, in step 2, the particles obtained in step 1 are collected by centrifugation at 1000 rpm for 3 minutes and resuspended in a solvent to thereby obtain particles having a dent.

In view of safety, examples of the solvent include, but are not limited to, aqueous media such as water, phosphate-buffered saline, and physiological saline, ethanol, and dimethyl sulfoxide, preferably aqueous media such as water and phosphate-buffered saline, and particularly preferably water.

The amount of solvent used for suspending the particles is not limited. For example, an aqueous medium is used in an amount such that the concentration of the particles in the suspension is 0.1 to 50 wt.%.

The weight ratio of the particles to the solvent (particles/solvent) is preferably in the range of 1/10000 to 3/2, and more preferably 1/1000 to 1/1, in terms of shape control.

### 4. Composition containing microparticle and organic solvent

As described above, a composition containing the microparticle of the present disclosure and an organic solvent is obtained in the process of producing the microparticle of the present disclosure.

### 5. Microparticle dispersion

The microparticle dispersion of the present disclosure is a microparticle dispersion comprising the microparticle of the present disclosure and an aqueous medium, the microparticle being dispersed in the aqueous medium.

The aqueous medium of the microparticle dispersion of the present disclosure can contain various additives.

The additives are not particularly limited as long as they can be dissolved or dispersed in an aqueous medium. Examples include buffers comprising inorganic salts, phosphate ions, and bicarbonate ions; amino acids; vitamins such as riboflavin, thiamine, and biotin; carbohydrates such as glucose, galactose, and maltose; peptides and proteins such as transferrin, fibronectin, and fetuin; fatty acids and lipids such as cholesterol and steroids; serum components such as albumin, growth factors, and growth inhibitors; and biological trace elements such as zinc, copper, and selenium.

The microparticle dispersion of the present disclosure can be used, for example, as a gas-carrying solution.

In the above, the gas-carrying solution can be, for example, an oxygen carrier. Specifically, the oxygen carrier can be used in applications such as blood substitutes, means for supplying oxygen to a specific site in the body, a graft tissue preservation solution, a transplanted organ protection solution, a transplanted organ perfusion solution, or an animal cell or microorganism culture solution.

When the microparticle dispersion of the present disclosure is to be used as a blood substitute, specific examples of applications include blood transfusion, prophylactic and/or therapeutic treatment of anoxic conditions (e.g., ischemia due to heart failure, cerebral infarction, respiratory failure, or the like), and prophylactic and/or therapeutic treatment of ischemic conditions or conditions following reperfusion, particularly tourniquet syndrome (reperfusion syndrome). "Anoxic conditions" are associated with ischemic and hypoxic conditions. "Ischemic" or "ischemia" refers to a reduction or cessation of blood flow to a tissue or organ.

When the microparticle dispersion of the present disclosure is to be used for transfusion, more specific examples of applications include the treatment of blood loss associated with surgery, accidents, injuries, etc. (e.g., hemorrhagic shock and bleeding during surgery).

When the microparticle dispersion of the present disclosure is to be used for graft tissue preservation, a specific example of application is an artificial oxygen carrier for transporting oxygen to the transplanted tissue. In this case, the microparticle dispersion of the present disclosure can also be used for graft tissue preservation in the field of regenerative medicine.

When the microparticle dispersion of the present disclosure is to be used as a non-oxygen gas-carrying solution, specific examples of applications include its use as a DDS carrier or the like for delivering a gas. In this case, the microparticle dispersion of the present disclosure can also be used for prophylactic and/or therapeutic treatment of gas bubble formation or gas thrombosis in the bloodstream (e.g., supplementation of an extracorporeal circulation circuit such as an artificial heart-lung machine, liquid ventilation, and detoxification).

When animals are targeted in the above applications, the targeted subject is not limited, and can be, for example, humans, mice, rats, rabbits, pigs, dogs, or cats.

### Examples

The present disclosure is described with reference to Examples. However, the present disclosure is not limited to these Examples. The unit in the tables represents mass.

### (1) Measurement of Size and Particle Size Distribution

The particles obtained in the Examples were subjected to measurements using a laser diffraction/scattering particle size distribution analyzer (Partica LA-950V2, Horiba) to determine the particle size (volume average particle diameter) and the CV value of the particles. The CV value was calculated from the standard deviation of the particle diameter and the average particle diameter according to the following formula: $CV value \left(%\right) = \left(Standard deviation of particle diameter/Average particle diameter\right) \times 100$

### (2) Evaluation of Elastic Modulus

The deformability of the microparticle was measured using a micro compression tester (MCT series, produced by Shimadzu Corporation). One measurement target was selected from the particles on the stage under microscopic observation (50x objective lens), and the particles were compressed from above with a probe to evaluate the stress and displacement during compression.

### I. Preparation of Microparticles

### 1. Spherical Microparticles

### Example 1

### Step 1:

30 mg of a tetrafluoroethylene-vinylpyrrolidone copolymer (weight average molecular weight: 44500, tetrafluoroethylene: vinylpyrrolidone = 36:64, fluorine content: 26 wt.%) and 0.12 g of perfluorooctyl bromide (oxygen solubility: 50 vol.%) were dissolved in 2 mL of dichloromethane. Nile red was added for fluorescence measurement. Subsequently, the resulting solution was subjected to membrane emulsification by extruding the solution into 20 mL of a 2% polyvinyl alcohol solution via a hydrophilic glass porous membrane with a syringe to obtain emulsion particles.

### Step 2:

Subsequently, dichloromethane was evaporated at room temperature while the emulsion was stirred at 200 rpm, and the resulting emulsion was further centrifuged at 1000 rpm for 3 minutes. The obtained particles were washed with pure water to obtain particles each composed of a core comprising perfluorooctyl bromide and a shell comprising a tetrafluoroethylene-vinylpyrrolidone copolymer. The particles had a volume average particle diameter of 11.93 µm and a CV value of 34%.

### Example 2

Particles composed of a core comprising perfluorooctyl bromide and a shell comprising a tetrafluoroethylene-vinylpyrrolidone copolymer were obtained in the same manner as in Example 1 except that 50 mg of the tetrafluoroethylene-vinylpyrrolidone copolymer was used. The particles had a volume average particle diameter of 10.97 µm and a CV value of 43%.

### Example 3

Particles composed of a core comprising perfluorooctyl bromide and a shell composed of a tetrafluoroethylene-vinylpyrrolidone copolymer were obtained in the same manner as in Example 1 except that 100 mg of a tetrafluoroethylene-vinylpyrrolidone copolymer was used. The particles had a volume average particle diameter of 12.37 µm and a CV value of 31%.

### Example 4

Emulsified particles were obtained in the same manner as in Example 1 except that 50 mg of poly(methyl α-fluoroacrylate) (weight average molecular weight: 10400, fluorine content: 18 wt.%) was used in step 1. In step 2, the solvent was then removed by evaporation and washing was performed to obtain particles composed of a core comprising perfluorooctyl bromide and a shell comprising poly(methyl α-fluoroacrylate). The particles had a volume average particle diameter of 7.57 µm and a CV value of 30%.

### Example 5

Emulsified particles were obtained in the same manner as in Example 1 except that 50 mg of an alternating copolymer of 4,4'-[perfluoropropane-2,2'-diyl]diphthalic anhydride and 2,2'-bis(trifluoromethyl)-4,4'diaminobiphenyl (fluorine content: 33% by weight) was used in step 1. In step 2, the solvent was then removed by evaporation and washing was performed to obtain particles composed of a core comprising perfluorooctyl bromide and a shell comprising an alternating copolymer of 4,4'-[perfluoropropane-2,2'-diyl]diphthalic anhydride and 2,2'-bis(trifluoromethyl)-4,4'diaminobiphenyl. The particles had a volume average particle diameter of 6.63 µm and a CV value of 29%.

### Example 6

Emulsified particles were obtained in the same manner as in Example 1 except that 50 mg of a polydimethylsiloxane elastomer (Product No.: Super-PDMS XM-1700) was used in step 1. In step 2, the solvent was then removed by evaporation and washing was performed to obtain particles composed of a core comprising perfluorooctyl bromide and a shell comprising polydimethylsiloxane. The particles had a volume average particle diameter of 10.1 µm and a CV value of 28%.

### Example 7

Emulsified particles were prepared in the same manner as in Example 1 except that 50 mg of a styrene-butadiene-styrene block copolymer (styrene: 30 wt.%, melt index: 10 g/10 min (200°C/5.0 kg) was used in step 1. In step 2, the solvent was then removed by evaporation and washing was performed to obtain particles composed of a core comprising perfluorooctyl bromide and a shell comprising a styrene-butadiene-styrene block copolymer. The particles had a volume average particle diameter of 9.3 µm and a CV value of 33%.

### Example 8

Emulsified particles were obtained in the same manner as in Example 1 except that 50 mg of a vinylidenefluoride-tetrafluoroethylene-hexafluoropropylene copolymer (content ratio: vinylidenefluoride/tetrafluoroethylene/hexafluoropropylene = 50/20/30, fluorine content 67 wt.%), 61 µL of perfluorodecalin (oxygen solubility: 40 vol.%), and hydrofluoroether (trade name: Novec 7000) as a solvent were used in step 1. In step 2, the solvent was then removed by evaporation and washing was performed to obtain particles composed of a core comprising perfluorodecalin and a shell comprising a vinylidenefluoride-tetrafluoroethylene-hexafluoropropylene copolymer. The particles had a volume average particle diameter of 18.62 µm and a CV value of 66%.

### Example 9

Emulsified particles were obtained in the same manner as in Example 1 except that 50 mg of a vinylidenefluoride-tetrafluoroethylene-hexafluoropropylene copolymer (content ratio: vinylidenefluoride/tetrafluoroethylene/hexafluoropropylene = 50/20/30, fluorine content: 67 wt.%), 61 µL of perfluoropolyether oil (Krytox GPL 103), and hydrofluoroether (trade name: Novec 7000) as a solvent were used in step 1. In step 2, the solvent was then removed by evaporation and washing was performed to obtain particles composed of a core comprising perfluoropolyether oil and a shell comprising a vinylidenefluoride-tetrafluoroethylene-hexafluoropropylene copolymer. The particles had a volume average particle diameter of 5.99 µm and a CV value of 34%.

### Example 10

Emulsified particles were obtained in the same manner as in Example 1 except that 50 mg of a vinylidenefluoride-tetrafluoroethylene-hexafluoropropylene copolymer (content ratio: vinylidenefluoride/tetrafluoroethylene/hexafluoropropylene = 50/20/30, fluorine content: 67 wt.%), 61 µL of perfluorooctyl bromide, and 2 mL of hydrofluoroether (trade name: Novec 7000) were used in step 1.

In step 2, the solvent was removed by evaporation and washing was performed to obtain particles composed of a core comprising of perfluorooctyl bromide and a shell comprising a vinylidenefluoride-tetrafluoroethylene-hexafluoropropylene copolymer. The particles had a volume average particle diameter of 6.31 µm and a CV value of 28%.

### Example 11

Emulsified particles were obtained in the same manner as in Example 1 except that 5 mg of an ethylene tetrafluoride-vinyl pyrrolidone copolymer and 45 mg of an alternating copolymer of 4,4'-[perfluoropropane-2,2-diyl]diphthalic anhydride and 2,2'-bis(trifluoromethyl)-4,4'diaminobiphenyl were used in step 1. In step 2, the solvent was then removed by evaporation and washing was performed to obtain particles composed of a core comprising perfluorodecalin and a shell comprising an alternating copolymer of 4,4'-[perfluoropropane-2,2'-diyl]diphthalic anhydride and 2,2'-bis(trifluoromethyl)-4,4'diaminobiphenyl. The particles had a volume average particle diameter of 5.86 µm and a CV value of 30%.

### Example 12

Emulsified particles were obtained in the same manner as in Example 1 except that 50 mg of an alternating copolymer of 4,4'-[perfluoropropane-2,2'-diyl]diphthalic anhydride and 2,2'-bis(trifluoromethyl)-4,4'diaminobiphenyl and a mixed solvent of dichloromethane and Novec 7000 (dichloromethane/Novec 7000 = 2 mL/0.2 mL) as a solvent were used in step 1. In step 2, the solvent was then removed by evaporation and washing was performed to obtain particles composed of a core comprising perfluorodecalin and a shell comprising an alternating copolymer of 4,4'-[perfluoropropane-2,2'-diyl]diphthalic anhydride and 2,2'-bis(trifluoromethyl)-4,4'diaminobiphenyl. The particles had a volume average particle diameter of 10.54 µm and a CV value of 29%.

### Example 13

Emulsified particles were obtained in the same manner as in Example 1 except that 50 mg of an alternating copolymer of 4,4'-[perfluoropropane-2,2'-diyl]diphthalic anhydride and 2,2'-bis(trifluoromethyl)-4,4'diaminobiphenyl, 58 µL of perfluorooctylbromide, and 4 µL of perfluorooctyldecane were used in step 1. In step 2, the solvent was then removed by evaporation and washing was performed to obtain particles composed of a core comprising perfluorooctylbromide and perfluorooctyldecane and a shell comprising an alternating copolymer of 4,4'-[perfluoropropane-2,2'-diyl]diphthalic anhydride/2,2'-bis(trifluoromethyl)-4,4'diaminobiphenyl. The particles had a volume average particle diameter of 6.7 µm and a CV value of 29%.

### Example 14

Emulsified particles were obtained in the same manner as in Example 1 except that 5 mg of a tetrafluoroethylene-vinylpyrrolidone copolymer, 45 mg of an alternating copolymer of 4,4'-[perfluoropropane-2,2'-diyl]diphthalic anhydride and 2,2'-bis(trifluoromethyl)-4,4'diaminobiphenyl, 61 µL of perfluorodecalin, and a mixed solvent of dichloromethane and Novec 7000 (dichloromethane/Novec 7000 = 2 mL/0.2 mL) as a solvent were used in step 1. In step 2, the solvent was then removed by evaporation and washing was performed to obtain particles composed of a core comprising perfluorodecalin and a shell comprising a tetrafluoroethylene-vinylpyrrolidone copolymer and an alternating copolymer of a 4,4'-[perfluoropropane-2,2'-diyl]diphthalic anhydride and 2,2'-bis(trifluoromethyl)-4,4'diaminobiphenyl. The particles had a volume average particle diameter of 28.9 µm and a CV value of 66%.

### Example 15

Emulsified particles were obtained in the same manner as in Example 1 except that 25 mg of a styrene-maleic anhydride copolymer and 25 mg of an alternating copolymer of 4,4'-[perfluoropropane-2,2'-diyl]diphthalic anhydride and 2,2'-bis (trifluoromethyl)-4,4'diaminobiphenyl were used in step 1. In step 2, the solvent was then removed by evaporation and washing was performed to obtain particles composed of a core comprising perfluorooctyl bromide and a shell comprising a styrene-maleic anhydride copolymer and an alternating copolymer of 4,4'-[perfluoropropane-2,2'-diyl]diphthalic anhydride and 2,2'-bis(trifluoromethyl)-4,4'-diaminobiphenyl. The particles had a volume average particle diameter of 6.58 µm and a CV value of 29%.

### Example 16

Emulsified particles were obtained in the same manner as in Example 1 except that 50 mg of poly[4,5-difluoro-2,2-bis(trifluoromethyl)-1,3-dioxol-co-tetrafluoroethylene], 61 µL of perfluoropolyether oil (Krytox GPL 103), and hydrofluoroether (trade name: Novec 7000) as a solvent were used in step 1. The obtained particles had a volume average particle diameter of 27.2 µm.

### 2. Microparticle having dent

### Example 17: Microparticle having dent

1 mL of isopropanol was added with stirring to 2 mL of a 0.8 wt.% dispersion of particles prepared in the same manner as in Example 5. The mixture was allowed to stand at room temperature for about 12 hours while being stirred at a rate at which the microparticle did not precipitate. The obtained particles were resuspended in pure water to obtain microparticles having a dent.

### Example 18

The particles produced in the same manner as in Example 6 were exposed to the following conditions for 24 hours: a dilute aqueous hydrochloric acid solution (pH = 4.4), an aqueous sodium hydroxide solution (pH = 10.4), 70% ethanol, and 120°C highpressure steam. After the exposure, the particles were observed with a scanning electron microscope and the core-shell particle shape was confirmed to have no changes.

### Example 19

The particles produced in the same manner as in Example 5 were exposed to the following conditions for 24 hours: a dilute aqueous hydrochloric acid solution (pH = 4.4), a sodium hydroxide solution (pH = 10.4), and 70% ethanol. After the exposure, the particles were observed with a scanning electron microscope, and the core-shell particle shape was confirmed to have no changes.

### Comparative Example 1

Core-shell type particles composed of a core comprising perfluorooctyl bromide and a shell comprising an L-lactic acid-caprolactone copolymer as described in Adv. Mater. Technol. 2021, 2100573 was immersed in an aqueous sodium hydroxide solution (pH = 10.4) for 24 hours. After the immersion, the particles were observed with a scanning electron microscope, and it was confirmed that the particle shape had changed and that the core-shell structure collapsed.

### Description of the Reference Numerals

- 1:: Microparticle
- 11:: Shell
- 12:: Core
- 13:: Polymer
- 14:: Fluorocarbon
- 15:: Dent
- da:: Short diameter
- db:: Long diameter

## Claims

1. A core-shell microparticle comprising
- a shell comprising at least one polymer selected from fluorine-containing polymers, fluorine-free vinyl polymers, and silicone polymers, and
- a core comprising a fluorocarbon, wherein the fluorocarbon is in liquid or gel form.

2. The microparticle of claim 1, wherein the structural unit constituting the main chain in the repeating unit in the main chain of the polymer substantially comprises at least one structural unit selected from -CX¹X²-CX³CX⁴-, -CX¹=CX²-, -CX¹-O-CX²-, -CX¹X²-C(=O) -CX³X⁴-, -C(=O)-N(X,Y)-, -CX¹X²-C(=S)-CX¹X²-, -Si(X,Y)-O-Si(X,Y)-, and -Si(X,Y)-Si(X,Y)-, wherein
X¹-X⁴ each independently are H, F, Cl, nitrile or ester, optionally substituted oxygen, or chain or cyclic C₁₋₂₀-alkyl, C₁₋₂₀-alkylene, C₁₋₂₀-fluoroalkyl or C₁₋₂₀-fluoroalkylene, each optionally substituted; and
X, Y each independently are H or a chain or cyclic group selected from C₁₋₂₀-alkyl, C₁₋₂₀-alkylene, C₁₋₂₀-aryl, C₁₋₂₀- (per) fluoroalkyl and C₁₋₂₀-(per) fluoroalkylene, each optionally substituted.

3. The microparticle of claim 1 or 2, wherein the polymer is a silicone polymer or a fluorine-containing polymer, and the fluorine content in the polymer is ≥ 20 wt%.

4. The microparticle of any of claims 1-3, wherein the fluorocarbon in the core
- has a fluorine content of ≥ 10 mass%, and/or
- has a boiling point of ≥ 90°C, and/or
- has an oxygen solubility of ≥ 30 vol/vol% at 25 °C.

5. The microparticle of any of claims 1-4, wherein the fluorocarbon has a weight average molecular weight of ≤ 50,000 and is
(i) a fluorocarbon containing a perfluoroalkyl group and optionally fluoropolyether group or
(ii) a fluorocarbon containing a perfluoroalkyl group and a fluoropolyether group, which preferably has a boiling point of ≥ 150°C,
wherein the weight average molecular weight is measured by gel permeation chromatography.

6. The microparticle of any of claims 1-5, having a volume average particle diameter of ≤ 500 µm, wherein the volume average particle diameter is measured by a scanning electron microscope image or using a laser diffraction/scattering particle size distribution analyzer.

7. The microparticle of any of claims 1-6, wherein the shell has a thickness of ≥ 50 nm, wherein the thickness of the shell is measured by a confocal microscope or an electron microscope.

8. The microparticle of any of claims 1-6, wherein the core contains oxygen.

9. The microparticle of any of claims 1-8, wherein the shell has an elastic modulus of ≤ 500 MPa, measured as specified in the description.

10. A composition comprising the microparticle of any of claims 1-9 and an organic solvent.

11. A microparticle dispersion comprising the microparticle of any of claims 1-9 dispersed in an aqueous medium.

12. The use of the microparticle dispersion of claim 11 as an oxygen-carrying solution, a transplanted tissue preservation solution, a transplanted organ protection solution, or a culture solution.

13. A method for producing the microparticle of any of claims 1-9, comprising the steps of
(1) subjecting a dispersed phase in which the polymer and the fluorocarbon are dispersed in an organic solvent to membrane emulsification in a continuous phase containing a surfactant via a porous membrane having a uniform pore diameter, thus obtaining an emulsion containing the microparticle as a dispersoid, and
(2) removing an organic solvent phase contained in the emulsion.

14. A method for producing the microparticle of any of claims 1-7 having a dent, comprising the steps of
(1) adding an alcohol to a dispersion obtained by dispersing the microparticle of any of claims 1-10 in an aqueous medium, and
(2) resuspending the particle obtained in step (1) in a solvent.

## Patentansprüche

1. Kern-Schale-Mikropartikel, umfassend
- eine Schale, umfassend mindestens ein Polymer, ausgewählt aus fluorhaltigen Polymeren, fluorfreien Vinylpolymeren und Silikonpolymeren, und
- einen Kern, umfassend einen Fluorkohlenwasserstoff, worin der Fluorkohlenwasserstoff in flüssiger oder gelartiger Form vorliegt.

2. Mikropartikel gemäß Anspruch 1, worin die Struktureinheit, die die Hauptkette in der Wiederholungseinheit der Hauptkette des Polymers bildet, im Wesentlichen mindestens eine Struktureinheit umfasst, ausgewählt aus -CX¹X²-CX³CX⁴-, -CX¹=CX²-, -CX¹-O-CX²-, - CX¹X²-C(=O)-CX³X⁴-, -C(=O)-N(X,Y)-, -CX¹X²-C(=S)-CX¹X²-, - Si(X,Y)-O-Si(X,Y)- und -Si(X,Y)-Si(X,Y)-, worin
X¹-X⁴ jeweils unabhängig voneinander H, F, Cl, Nitril oder Ester, gegebenenfalls substituierter Sauerstoff oder ein kettenförmiges oder cyclisches C₁₋₂₀-Alkyl, C₁₋₂₀-Alkylen, C₁₋₂₀-Fluoralkyl oder C₁₋₂₀-Fluoralkylen, jeweils gegebenenfalls substituiert, sind; und
X, Y jeweils unabhängig voneinander H oder eine kettenförmige oder cyclische Gruppe, ausgewählt aus C₁₋₂₀-Alkyl, C₁₋₂₀-Alkylen, C₁₋₂₀-Aryl, C₁₋₂₀-(Per)fluoralkyl und C₁₋₂₀-(Per)fluoralkylen, jeweils gegebenenfalls substituiert, sind.

3. Mikropartikel gemäß Anspruch 1 oder 2, worin das Polymer ein Silikonpolymer oder ein fluorhaltiges Polymer ist und der Fluorgehalt im Polymer ≥ 20 Gew.-% beträgt.

4. Mikropartikel gemäß einem der Ansprüche 1-3, worin der Fluorkohlenwasserstoff im Kern
- einen Fluorgehalt von ≥ 10 Massen-% aufweist und/oder
- einen Siedepunkt von ≥ 90 °C aufweist und/oder
- eine Sauerstofflöslichkeit von ≥ 30 Vol/Vol.-% bei 25 °C aufweist.

5. Mikropartikel gemäß einem der Ansprüche 1-4, worin der Fluorkohlenwasserstoff ein gewichtsmittleres Molekulargewicht von ≤ 50.000 aufweist und
(i) ein Fluorkohlenwasserstoff, der eine Perfluoralkylgruppe und gegebenenfalls eine Fluorpolyethergruppe enthält, oder
(ii) ein Fluorkohlenwasserstoff ist, der eine Perfluoralkylgruppe und eine Fluorpolyethergruppe enthält, und vorzugsweise einen Siedepunkt von ≥ 150 °C aufweist,
worin das gewichtsmittlere Molekulargewicht mittels Gelpermeationschromatographie bestimmt wird.

6. Mikropartikel gemäß einem der Ansprüche 1-5, der einen volumenmittleren Partikeldurchmesser von ≤ 500 µm aufweist, worin der volumenmittlere Partikeldurchmesser anhand einer Rasterelektronenmikroskop-Aufnahme oder unter Verwendung eines Analysators zur Bestimmung der Partikelgrößenverteilung mittels Laserbeugung/-streuung gemessen wird.

7. Mikropartikel gemäß einem der Ansprüche 1-6, worin die Hülle eine Dicke von ≥ 50 nm aufweist, worin die Dicke der Hülle mittels eines konfokalen Mikroskops oder eines Elektronenmikroskops gemessen wird.

8. Mikropartikel nach einem der Ansprüche 1-6, worin der Kern Sauerstoff enthält.

9. Mikropartikel gemäß einem der Ansprüche 1-8, worin die Hülle ein Elastizitätsmodul von ≤ 500 MPa aufweist, gemessen wie in der Beschreibung angegeben.

10. Zusammensetzung, umfassend den Mikropartikel gemäß einem der Ansprüche 1-9 und ein organisches Lösungsmittel.

11. Mikropartikeldispersion, umfassend den Mikropartikel gemäß einem der Ansprüche 1-9, dispergiert in einem wässrigen Medium.

12. Verwendung der Mikropartikeldispersion gemäß Anspruch 11 als sauerstofftragende Lösung, als Lösung zur Konservierung von Transplantatgewebe, als Lösung zum Schutz von Transplantatorganen oder als Kulturlösung.

13. Verfahren zur Herstellung des Mikropartikels gemäß einem der Ansprüche 1-9, umfassend die Schritte
(1) Unterziehen einer dispergierten Phase, in der das Polymer und der Fluorkohlenwasserstoff in einem organischen Lösungsmittel dispergiert sind, einer Membranemulgierung in einer ein Tensid enthaltenden kontinuierlichen Phase durch eine poröse Membran mit gleichmäßigem Porendurchmesser, wodurch eine Emulsion erhalten wird, die den Mikropartikel als Dispersoid enthält, und
(2) Entfernen einer in der Emulsion enthaltenen organischen Lösungsmittelphase.

14. Verfahren zur Herstellung des Mikropartikels gemäß einem der Ansprüche 1-7, der eine Vertiefung aufweist, umfassend die Schritte
(1) Zugeben eines Alkohols zu einer Dispersion, die durch Dispergieren des Mikropartikels gemäß einem der Ansprüche 1-10 in einem wässrigen Medium erhalten wurde, und
(2) Resuspendieren des in Schritt (1) erhaltenen Partikels in einem Lösungsmittel.

## Revendications

1. Microparticule à noyau et enveloppe comprenant
- une enveloppe comprenant au moins un polymère sélectionné parmi des polymères contenant du fluor, des polymères vinyliques exempts de fluor et des polymères de silicone, et
- un noyau comprenant un fluorocarbure, dans laquelle le fluorocarbure est sous forme liquide ou de gel.

2. Microparticule selon la revendication 1, dans laquelle l'unité structurelle constituant la chaîne principale dans l'unité répétitive dans la chaîne principale du polymère comprend sensiblement au moins une unité structurelle sélectionnée parmi -CX¹X²-CX³CX⁴-, -CX¹=CX²-, -CX¹-O-CX²-, -CX¹X²-C(=O)-CX³X⁴-, -C(=O)-N(X,Y)-, -CX¹X²-C(=S)-CX¹X²-, -Si(X,Y)-O-Si(X,Y)- et -Si(X,Y)-Si(X,Y)-, dans laquelle
X¹-X⁴ sont chacun indépendamment H, F, CI, nitrile ou ester, oxygène substitué facultativement, ou C₁₋₂₀-alkyle, C₁₋₂₀-alkylène, C₁₋₂₀-fluoroalkyle ou C₁₋₂₀-fluoroalkylène linéaire ou cyclique, chacun étant substitué facultativement ; et
X, Y sont chacun indépendamment H ou un groupe linéaire ou cyclique sélectionné parmi C₁₋₂₀-alkyle, C₁₋₂₀-alkylène, C₁₋₂₀-aryle, C₁₋₂₀-(per)fluoroalkyle et C₁₋₂₀-(per)fluoroalkylène, chacun étant substitué facultativement.

3. Microparticule selon la revendication 1 ou la revendication 2, dans laquelle le polymère est un polymère de silicone ou un polymère contenant du fluor, et la teneur en fluor dans le polymère est ≥ 20 % en poids.

4. Microparticule selon l'une quelconque des revendications 1 à 3, dans laquelle le fluorocarbure dans le noyau
- présente une teneur en fluor de ≥ 10 % en masse, et/ou
- présente un point d'ébullition de ≥ 90 °C, et/ou
- présente une solubilité en oxygène de ≥ 30 % vol/vol à 25 °C.

5. Microparticule selon l'une quelconque des revendications 1 à 4, dans laquelle le fluorocarbure présente une masse moléculaire moyenne en poids de ≤ 50 000 et est
(i) un fluorocarbure contenant un groupe perfluoroalkyle et facultativement un groupe fluoropolyéther ou
(ii) un fluorocarbure contenant un groupe perfluoroalkyle et un groupe fluoropolyéther, qui présente préférablement un point d'ébullition de ≥ 150 °C,
dans laquelle la masse moléculaire moyenne en poids est mesurée par chromatographie par perméation de gel.

6. Microparticule selon l'une quelconque des revendications 1 à 5, présentant un diamètre volumique moyen de particules de ≤ 500 µm, dans laquelle le diamètre volumique moyen de particules est mesuré par une image de microscope électronique à balayage ou en utilisant un analyseur de distribution de taille de particules par diffraction/diffusion laser.

7. Microparticule selon l'une quelconque des revendications 1 à 6, dans laquelle l'enveloppe présente une épaisseur de ≥ 50 nm, dans laquelle l'épaisseur de l'enveloppe est mesurée par un microscope confocal ou un microscope électronique.

8. Microparticule selon l'une quelconque des revendications 1 à 6, dans laquelle le noyau contient de l'oxygène.

9. Microparticule selon l'une quelconque des revendications 1 à 8, dans laquelle l'enveloppe présente un module d'élasticité de ≤ 500 MPa, mesuré comme spécifié dans la description.

10. Composition comprenant la microparticule selon l'une quelconque des revendications 1 à 9 et un solvant organique.

11. Dispersion de microparticules comprenant la microparticule selon l'une quelconque des revendications 1 à 9 dispersée dans un milieu aqueux.

12. Utilisation de la dispersion de microparticules selon la revendication 11 comme solution porteuse d'oxygène, solution de préservation de tissu transplanté, solution de protection d'organe transplanté, ou solution de culture.

13. Procédé de production de la microparticule selon l'une quelconque des revendications 1 à 9, comprenant les étapes consistant à
(1) soumettre une phase dispersée dans laquelle le polymère et le fluorocarbure sont dispersés dans un solvant organique à une émulsification sur membrane dans une phase continue contenant un agent tensioactif via une membrane poreuse présentant un diamètre de pores uniforme, obtenant ainsi une émulsion contenant la microparticule en tant que dispersoïde, et
(2) éliminer une phase de solvant organique contenue dans l'émulsion.

14. Procédé de production de la microparticule selon l'une quelconque des revendications 1 à 7 présentant un creux, comprenant les étapes consistant à
(1) ajouter un alcool à une dispersion obtenue par dispersion de la microparticule selon l'une quelconque des revendications 1 à 10 dans un milieu aqueux, et
(2) remettre en suspension la particule obtenue à l'étape (1) dans un solvant.
